# EUROPEAN PATENT APPLICATION

(11) **EP 1 445 612 A1**
(43) Date of publication of application: **11.08.2004**
(21) Application number: 02777814.1
(22) Date of filing: 08.10.2002
(51) Int. Cl.: G01N 33/50, A61K 45/00, A61P 35/00, A61P 43/00, G01N 33/15

(54) **NOVEL METHOD OF ASSAYING IMMUNE ACTIVITY**

(30) Priority: 09.10.2001 JP 2001312051
(71) Applicant: Orient Cancer Therapy Co. Ltd, Mitaka-shi, Tokyo 181-0015 (JP)
(72) Inventor: YAGITA, Akikuni, Mitaka-shi, Tokyo 181-0015 (JP)
(74) Representative: Krauss, Jan
(86) International application number: PCT/JP2002/010448
(87) International publication number: WO 2003/031969

(57) **Abstract**

To provide a novel method for assay of CTL activity, NKT activity and NK activity, to analyze the meaning of the test of each of the assayed results using the assay method and to find each of the significances of CTL activity, NKT activity and NK activity in cancer therapy. To be specific, the present invention has succeeded in providing a novel method for assay of CTL activity, NKT activity and NK activity, analyzed the meaning of the test of each of the assayed results using the assay method and found each of the significances of CTL activity, NKT activity and NK activity in cancer therapy whereupon a novel immunotherapy comprising the present invention has been achieved.

## Description

The present invention claims a priority on the Japanese Patent Application 2001/312,051 which is cited here by reference.

### Technical Field

The present invention relates to providing a novel means for new immunotherapy where attention is paid to the kinetics of lymphocytes in peripheral blood. To be more specific, it relates to a method for assaying immune function of human being in a new immunotherapy comprising a test of correlation of CTL activity, NK activity and NKT activity to the kinetics of lymphocytes in peripheral blood.

### Background of the Invention

For the selection of a substance which is useful for prevention or therapy of cancer or malignant neoplasms, its direct action to cancer cells has been considered to be important. Although immunopotentiators have been recognized to be useful for the therapy of cancer, anti-cancer effect of all compounds which are prepared as immunopotentiators is weak and, even by a sole immunotherapy or by a joint therapy with chemotherapy, a sufficient therapeutic effect for cancer has not been achieved.

Dr. Yagita who is the inventor of the present invention has previously paid his attention to the usefulness of a substance which induces interleukin 12 (IL-12) *in vivo* as an epoch-making means in the therapy of cancer, found a processed product of mycelia of *shiitake* (*Lentinus edodes*) has such a function and established a method for the therapy of cancer which may be said as a novel immunotherapy for cancer (NITC). Until that time, there was a fact that, although IL-12 has an anti-cancer effect, it results in a side effect when IL-12 per se is directly administered into living body whereby the substance *per se* was unable to be used as an anti-cancer agent. However, a preparation containing the processed product of mycelia of *shiitake* reported by Yagita achieved a significant curing and life-supporting effect. Thus, Yagita achieved an object of therapy of cancer by administration of an effective amount of processed product of mycelia of *shiitake* which was able to induce IL-12 *in vivo* (Japanese Patent Laid-Open No. 10/139,670).

IL-12 has an activating effect and a potentiating effect for killer T cells by a route of TNFα → IFNγ → IL-12 → CTL activity. Thus, potentiation of production of IL-12 gives activating and potentiating effects for killer T cells and, as a result, anti-cancer effect is expected.

Yagita reported that, besides the system of potentiation of production of IL-12, activation of NKT cells is useful as the anti-cancer effect. Taniguchi, et al. found a specific glycolipid antigen which is recognized by Vα24Vβ11 which is a specific T cell antigen receptor (TCR) owned by NKT cells and reported that the antigen is α-galactosylceramide. It was further proved that, in a cancer-bearing mouse to which α-galactosylceramide was administered, NKT cells were activated and, although disappearance of cancer was not noted, metastasis was suppressed.

It was reported that, in NKT cells, there is an NK cell antigen receptor (NKT-P1; natural killer receptor P1) as another receptor (Special Issue for Ground and Clinic of NKT Cell: *Saishin Igaku,* volume 55, no. 4, pages 818-813, 2000). Yagita has found that NKT-P1 also participates in activation of NKT cells and the activation as such has a more dominant anti-cancer effect.

It was reported that NK cells also participate in anti-cancer action for living body but, up to now, there is no correlation between activity of NK cells and its clinical anti-cancer effect and the fact that induced production amount of IL-12 and activity of NK cell are in an entirely inverse correlation has been proved by Yagita whereby participation of NK cell in an anti-cancer action in human being has been questioned.

### Disclosure of the Invention

An object of the present invention is to provide a novel method for assay of CTL activity, NKT activity and NK activity as mentioned above, to analyze the meanings of the calibration of each of the assayed results using the assay method and to find each of the meanings of CTL activity, NKT activity and NK activity in cancer therapy whereupon a novel immunotherapy is completed.

The present invention succeeded in providing a novel method for the assay of CTL activity, NKT activity and NK activity, analyzed the meaning of calibration of each assayed result using the assay method, newly found each significance of CTL activity, NKT activity and NK activity in cancer therapy and achieved a novel immunotherapy comprising the present invention.

Thus, the present invention comprises the followings.
1. A method for an assay of the immune function of human being in a novel immunotherapy where at least one of the following assays is carried out for lymphocytes in peripheral blood:
   1) CD8(+)perforin productivity,
   2) CD3(-)CD161(+),
   3) CD3(-)CD161(+)perforin productivity,
   4) CD3(+)CD161(+),
   5) CD3(+)CD161(+)perforin productivity.
2. The method for the assay mentioned in the above 1, wherein the meaning of each assay is as follows:
   1) the function which is meant by the assay of CD8(+)perforin productivity is an assay of CTL activity,
   2) the function which is meant by the assay of CD3(-)CD161(+) is an assay of NK cell,
   3) the function which is meant by the assay of CD3(-)CD161(+)perforin productivity is an assay of activation of NK cell,
   4) the function which is meant by the assay of CD3(+)CD161(+) is an assay of NKT cell,
   5) the function which is meant by the assay of CD3(+)CD161(+) perforin productivity is an assay of activation of NKT cell.
3. A method for a screening of a CTL activator comprising an assay of CD8(+)perforin productivity.
4. A novel β-1,3-glucan bearing a CTL activating ability obtained by the method for the screening of the above 3.
5. A CTL activator containing β-1,3-glucan obtained by the method for the screening of the above 3.
6. A method for a screening of an NK activator comprising an assay of CD3(-)CD161(+)perforin productivity.
7. A novel α-1,3-glucan bearing an NK activating ability obtained by the method for the screening of the above 6.
8. An NK activator containing an α-1,3-glucan obtained by the method for the screening of the above 6.
9. A method for an assay of kinetics of NK cells and NKT cells, characterized in that, CD3(-)CD161(+), CD3(-)CD161(+)perforin productivity, CD3(+)CD161(+) and CD3 (+) CD161 (+) perforin productivity of the above 1 are assayed at the same time.
10. A method for a fractional screening for an NK activator or an NKT activator comprising the method of the above 9 where activations of the NK cells and NKT cells are in an inverse correlation.
11. A novel α-1,3-glucan bearing an NK activating ability or an NKT activating ability obtained by the method for the screening of the above 10.
12. An NK activator or an NKT activator containing an α-1,3-glucan obtained by the method for the screening of the above 10.
13. A method for a calibration of correlation between the cancer species by the method for the assay of the above 9 and kinetics of NK cells and NKT cells.
14. An NK cell activator containing α-1,3-glucan used for prostate cancer accompanied by the calibration of the above 13.
15. An NK cell activator containing α-1,3-glucan used for lung cancer accompanied by the calibration of the above 13.
16. An activator for NK cells or NKT cells containing α-1,3-glucan used in combination with at least one of anti-cancer agent, radioactivity and steroid therapy accompanied by the calibration of the above 13.
17. A commercial medium where the information mentioned in any of the above 1 to 16 is carried on a medium utilizing the law of nature.
18. A commercial method utilizing the commercial medium of the above 17.

### Brief Description of the Drawings

Fig. 1 is a drawing of all cases (103 cases) showing the correlation between induction of production of IL-12 and CD8(+)perforin(+).
Fig. 2 is a drawing of CR + PR cases (23 cases) showing the correlation between induction of production of IL-12 and CD8(+)perforin(+).
Fig. 3 is a drawing of CR + PR + LNC cases (31 cases) showing the correlation between induction of production of IL-12 and CD8(+)perforin(+).
Fig. 4 is a drawing of LNC cases (10 cases) showing the correlation between induction of production of IL-12 and CD8(+)perforin(+).
Fig. 5 is a drawing of SNC cases (31 cases) showing the correlation between induction of production of IL-12 and CD8(+)perforin(+).
Fig. 6 is a drawing of NC cases (41 cases) showing the correlation between induction of production of IL-12 and CD8(+)perforin(+).
Fig. 7 is a drawing of PD cases (10 cases) showing the correlation between induction of production of IL-12 and CD8(+)perforin(+).
Fig. 8 is a drawing of cases of the first medical examination (26 cases) showing the correlation between induction of production of IL-12 and CD8(+)perforin(+).
Fig. 9 is a drawing of all cases (103 cases) showing the correlation between induction of production of INFγ and CD8(+)perforin(+).
Fig. 10 is a drawing of CR + PR cases (26 cases) showing the correlation between induction of production of INFγ and CD8(+)perforin(+).
Fig. 11 is a drawing of CR + PR + LNC cases (36 cases) showing the correlation between induction of production of INFγ and CD8(+)perforin(+).
Fig. 12 is a drawing of LNC cases (10 cases) showing the correlation between induction of production of INFγ and CD8(+)perforin(+).
Fig. 13 is a drawing of SNC cases (31 cases) showing the correlation between induction of production of INFγ and CD8(+)perforin(+).
Fig. 14 is a drawing of NC cases (41 cases) showing the correlation between induction of production of INFγ and CD8(+)perforin(+).
Fig. 15 is a drawing of PD cases (10 cases) showing the correlation between induction of production of INFγ and CD8(+)perforin(+).
Fig. 16 is a drawing of cases of the first medical examination (26 cases) showing the correlation between induction of production of INFγ and CD8(+)perforin(+).
Fig. 17 is a drawing of all cases (98 cases) showing the correlation between NK cell activity and CD3 (-) CD161 (+) in the conventional method.
Fig. 18 is a drawing of CR cases (7 cases) showing the correlation between NK cell activity and CD3 (-) CD161 (+) in the conventional method.
Fig. 19 is a drawing of PR cases (6 cases) showing the correlation between NK cell activity and CD3(-)CD161(+) in the conventional method.
Fig. 20 is a drawing of CR + PR cases (13 cases) showing the correlation between NK cell activity and CD3 (-) CD161 (+) in the conventional method.
Fig. 21 is a drawing of LNC cases (15 cases) showing the correlation between NK cell activity and CD3 (-) CD161 (+) in the conventional method.
Fig. 22 is a drawing of SNC cases (13 cases) showing the correlation between NK cell activity and CD3 (-) CD161 (+) in the conventional method.
Fig. 23 is a drawing of NC cases (28 cases) showing the correlation between NK cell activity and CD3 (-) CD161 (+) in the conventional method.
Fig. 24 is a drawing of CR + PR + LNC cases (28 cases) showing the correlation between NK cell activity and CD3(-)CD161(+) in the conventional method.
Fig. 25 is a drawing of PD cases (24 cases) showing the correlation between NK cell activity and CD3(-)CD161(+) in the conventional method.
Fig. 26 is a drawing of cases of the first medical examination (33 cases) showing the correlation between NK cell activity and CD3(-)CD161(+) in the conventional method.
Fig. 27 is a drawing of all cases (98 cases) showing the correlation between NK cell activity and CD3(-)CD161(+)perforin(+) in the conventional method.
Fig. 28 is a drawing of CR + PR cases (13 cases) showing the correlation between NK cell activity and CD3(-)CD161(+)perforin(+) in the conventional method.
Fig. 29 is a drawing of CR + PR + LNC cases (28 cases) showing the correlation between NK cell activity and CD3(-)CD161(+)perforin(+) in the conventional method.
Fig. 30 is a drawing of NC cases (28 cases) showing the correlation between NK cell activity and CD3(-)CD161(+)perforin(+) in the conventional method.
Fig. 31 is a drawing of PD cases (24 cases) showing the correlation between NK cell activity and CD3(-)CD161(+)perforin(+) in the conventional method.
Fig. 32 is a drawing of cases of the first medical examination (33 cases) showing the correlation between NK cell activity and CD3(-)CD161(+)perforin(+) in the conventional method.
Fig. 33 is a drawing which shows variations in CD3(-)CD161(+) in the cases where TOG was administered. All cases (148 cases), CR + PR cases (45 cases) and PR cases (60 cases) are shown.
Fig. 34 is a drawing of all cases (98 cases) showing the correlation between CD3(+)CD161(+) and CD3(-)CD161(+).
Fig. 35 is a drawing of CR + PR cases (13 cases) showing the correlation between CD3(+)CD161(+) and CD3(-)CD161(+).
Fig. 36 is a drawing of CR + PR + LNC cases (28 cases) showing the correlation between CD3(+)CD161(+) and CD3(-)CD161(+).
Fig. 37 is a drawing of NC cases (28 cases) showing the correlation between CD3(+)CD161(+) and CD3(-)CD161(+).
Fig. 38 is a drawing of PD cases (24 cases) showing the correlation between CD3(+)CD161(+) and CD3(-)CD161(+).
Fig. 39 is a drawing of cases of the first medical examination (33 cases) showing the correlation between CD3(+)CD161(+) and CD3(-)CD161(+).
Fig. 40 is a drawing of all cases (98 cases) showing the correlation between NK cell activity and CD3(+)CD161(+)perforin(+) in the conventional method.
Fig. 41 is a drawing of CR + PR cases (13 cases) showing the correlation between NK cell activity and CD3(+)CD161(+)perforin(+) in the conventional method.
Fig. 42 is a drawing of CR + PR + LNC cases (28 cases) showing the correlation between NK cell activity and CD3(+)CD161(+)perforin(+) in the conventional method.
Fig. 43 is a drawing of NC cases (28 cases) showing the correlation between NK cell activity and CD3(+)CD161(+)perforin(+) in the conventional method.
Fig. 44 is a drawing of PD cases (24 cases) showing the correlation between NK cell activity and CD3(+)CD161(+)perforin(+) in the conventional method.
Fig. 45 is a drawing of cases of the first medical examination (33 cases) showing the correlation between NK cell activity and CD3(+)CD161(+)perforin(+) in the conventional method.
Fig. 46 is a drawing of all cases (98 cases) showing the correlation between CD3(+)CD161(+)perforin(+) and CD3(-)CD161(+)perforin(+).
Fig. 47 is a drawing of CR + PR cases (13 cases) showing the correlation between CD3(+)CD161(+)perforin(+) and CD3(-)CD161(+)perforin(+).
Fig. 48 is a drawing of CR + PR + LNC cases (28 cases) showing the correlation between CD3(+)CD161(+)perforin(+) and CD3(-)CD161(+)perforin(+).
Fig. 49 is a drawing of NC cases (28 cases) showing the correlation between CD3(+)CD161(+)perforin(+) and CD3(-)CD161(+)perforin(+).
Fig. 50 is a drawing of PD cases (24 cases) showing the correlation between CD3(+)CD161(+)perforin(+) and CD3(-)CD161(+)perforin(+).
Fig. 51 is a drawing of cases of the first medical examination (33 cases) showing the correlation between CD3(+)CD161(+)perforin(+) and CD3(-)CD161(+)perforin(+).
Fig. 52 is a drawing which shows the changes in CD3(+)CD161(+) (23 cases) as an influence of anti-cancer agent, radioactivity and steroid on immunological competence.
Fig. 53 is a drawing which shows the changes in CD3(-)C0161(+) (23 cases) as an influence of anti-cancer agent, radioactivity and steroid on immunological competence.
Fig. 54 is a drawing which shows the changes in induction of produced amount of INFγ (26 cases) as an influence of anti-cancer agent, radioactivity and steroid on immunological competence.
Fig. 55 is a drawing which shows the changes in inductin of produced amount of IL-12 (26 cases) as an influence of anti-cancer agent, radioactivity and steroid on immunological competence.

### Best Mode for Carrying Out the Invention

As hereunder, the present invention will be illustrated in detail and, unless otherwise defined, technical and scientific terms used in the present specification have the meanings which are understood in an usual manner by persons skilled in the art belonging to the present invention.

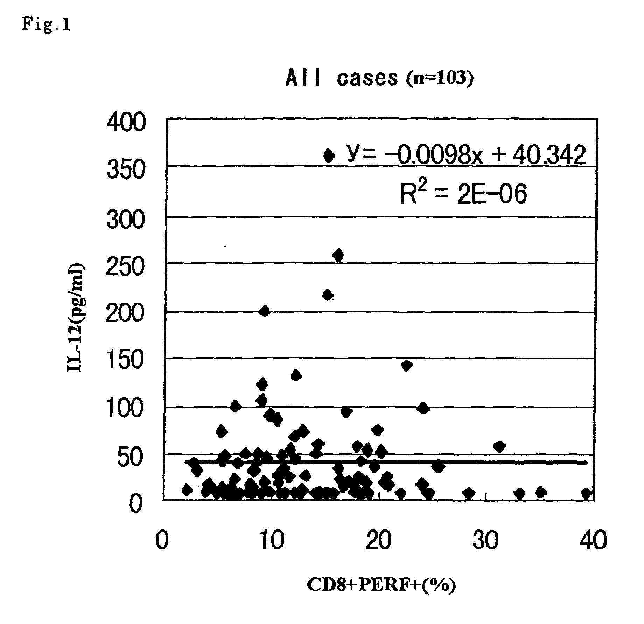

The present invention has been carried out by investigating the correlation between the clinical results and cytokine. As a novel immunotherapy for cancer (NITC), the present inventor has used a substance derived from mycelia of *shiitake* and a substance which inhibits angiogenesis (cartilage of shark) together in a patient suffering from a progressive terminal cancer and measured various markers for IL-12, IFN-γ, etc. and found that, in the cases where cancer is cured or does not worsen for a long period, there is a strongly positive correlation between the production of CD8(+)perforin and the production of IFN-γ and IL-12. As a result, it has been found that assay of the production of CD8(+)perforin has a significance in the evaluation of CTL activation route. However, CD8 (+) cell includes CTL (killer T cell) and suppressor T cell (immunosuppressive cell). Thus, with regard to the cell having CD8 which is a cell-surface marker, the above two kinds have been known. At the present stage, it is not possible to discriminate them by a cell-surface marker. That is also the same in the case of CD8(+)perforin but, as shown in Fig. 1 to Fig. 8, it has been found that, in the cases of the state where cancer is cured or does not worsen for a long period (CR + PR + and CR + PR + LNC in the drawings), it shows a positive correlation to IL-12 and IFN-γ of Th1 cytokine while, in the cases where cancer progresses or where the state of non-worsening of cancer is short (PD and SNC in the drawings), it shows a negative correlation thereto. Such a fact means the CD8(+)perforin cells in patients suffering from CR, PR and LNC are CTL. On the contrary, CD8(+)perforin cell observed in the cases of SNC and PD is believed to be a suppressor T cell. Thus, CD8 (+) or CD8(+)perforin in a state where Th1 cytokine exhibits an anti-tumor property or, to be specific, in the circumstance where 10 IU/ml or more IFN-γ is produced or 7.8 pg/ml or more IL-12 is produced is CTL while CD8 (+) or CD8(+)perforin in the circumstance where only 10 IU/ml or less IFN-γ is produced or only 7.8 pg/ml or less IL-12 is produced is a suppressor cell. Accordingly, it is important to assay IFN-γ and/or IL-12 for the assay of CD8(+) or CD8(+)perforin in the evaluation of CTL activation route.

As a result of finding the above-mentioned significance, it has been further found that the assay of CD8(+)perforin productivity is applicable to a method for screening of useful CTL activator and that, when such a screening method is utilized, it is possible to specify β-1,3-glucan bearing the CTL activating ability. In the present invention, it has been confirmed that preparations of *shiitake* mycelium composition having a β-1,3-glucan structure (such as ILX and ILY; K. K. Tozai Iyaku Kenkyusho; all being registered as commercial products) are effective for activation of CD8(+) perforin productivity and reconfirmed that they are effective for activation of CTL. Otherwise, various compounds having a β-1,3-glucan structure have been known and, when such a known structure is combined with an assay for CD8 perforin productivity, it is possible for persons skilled in the art to easily specify a CTL activator.

In the present invention, significance of NK cells has been newly established from the relation to NKT cells. For the establishment as such, the present inventor provided a novel method for the assay of NK cells. That has been found that an assay of CD3(-)CD161(+) is a correct NK cell assay as a result of comparison with the result of the known chromium release method. It has been also found that an assay of CD3(-)CD161(+)perforin productivity means activation of NK cells and further that cancer for which such an activation is effective is especially a prostate cancer. Thus, a method including an assay of CD3(-)CD161(+) perforin productivity is useful as a method for screening of NK activator. Moreover, in the present invention, it has been found that preparations of compositions such as nigerooligosaccharide and fucoidan having an α-1,3-glucan structure are effective for activation of CD3(-)CD161(+) perforin productivity and it has been reconfirmed that they are effective for activation of NK cells. The novel α-1,3-glucan bearing an NK activating ability obtained by the screening method is useful as an anti-cancer agent. Besides that, various compounds having an α-1,3-glucan structure have been known and, when such a known structure and assay of CD3 (-)CD161 (+) perforin productivity are combined, it is possible for persons skilled in the art to easily specify NK activators.

In the present invention, there has been also found a significance for a simultaneous assay of CD3(-)CD161(+), CD(-)CD161(+) perforin productivity, CD3(+)CD161(+) and CD3(+)CD161(+) perforin productivity and for conducting a method for assaying the kinetics of NK cells and NKT cells. Thus, the present inventor has conducted analysis utilizing the assay and, as a result, he has found that activation of both NK cells and NKT cells is effective by the above-mentioned compound having the α-1,3-glucan structure. NK cells and NKT cells have common NKR-P1 (CD161(+)) surface marker and have NKR-P1 receptor. CD161(+) receptor is activated by α-1,3-glucan. Accordingly, both NK cells and NKT cells are able to be activated by α-1,3-glucan. In cancer patients, NK cells are activated within 1 to 2 month(s) and then the activity lowers while NKT cells are activated within 3 to 4 months. It has been further found that activation of NK cells and NKT cells are in a negative correlation between both or, in other words, when NK cells are activated, then NKT cells are suppressed while, when NKT cells are activated, then NK cells are suppressed. As a result, both systems are in a complementary relation each other and it has been found that anti-cancer agent should be utilized after being selected depending upon type of cancer, adaptability to a patient such as state of immunological competence of a patient, stage of administration of anti-cancer agent, α-1,3-glucan structure use for the therapy, etc. In the present invention, the above-mentioned assay method is utilized whereupon it is possible to provide a screening method by which fractionation of NK activator or NKT activator including the above-mentioned assay method is made possible. It has been confirmed that, in the present invention, preparations of compositions such as nigerooligosaccharide, fucoidan and/or *Porphyra Yezaensis* represented by *Porphyra tenera* having an α-1,3-glucan structure are useful. Thus, a novel α-1,3-glucan bearing NK activating ability or NKT activating ability obtained by the screening method is useful as an anti-cancer agent. Besides that, various compounds having an α-1,3-glucan structure have been known and, when such a known structure is combined with assay of CD3(-)CD161(+), CD(-)CD161(+) perforin productivity, CD3(+)CD161(+) and CD3 (+) CD161 (+) perforin productivity, it is possible for persons skilled in the art to easily specify NK activator or NKT activator. Incidentally, CD161(+) means that it acts on NKR-P1 which is a common receptor for NK cells and NKT cells.

When correlation between the cancer species and the kinetics of NK cells and NKT cells was tested utilizing the assay method according to the present invention was investigated, it has been suggested that cancer species in which NK cells contribute to the cure therefor are prostate cancer, a part of lung cancer and a part of stomach cancer while cancer species in whicn NKT cells contribute the cure therefor are most of lung cancer and a part of stomach cancer. It has been also found that, as an intermediate type thereof, breast cancer and large intestine cancer are classified. It has been further confirmed that an NK cell activator containing α-1,3-glucan is useful specifically for prostate cancer, a part of lung cancer and a part of stomach cancer while an NKT cell activator containing α-1,3-glucan is useful specifically for most of lung cancer and a part of stomach cancer.

As mentioned above, when the assay method of the present invention is utilized either solely or jointly, immunological competence in a patient suffering from cancer is assayed and, when effective therapeutic method and drug and drug composition used for the therapy are selected is view of the assayed result and the cancer type, there is achieved far more effective therapeutic effect for cancer as compared with the conventional one.

In addition, it is believed to achieve the same result and effect as in the assay method of the present invention when characteristics of each cell being able to discriminate CTL, NK cells and NKT cells and gene related to various markers such as CD3, CD8, CD161, perforin, IL-12 and IFN-γ are assayed in view of the assay method of the present invention. Thus, an assay method according to the above-mentioned present invention by assaying such a gene, a screening method according to the above-mentioned present invention and a substance obtained by the screening method are also covered within a scope of the present invention.

Utilizing the assay method of the present invention, the present inventor has further confirmed the influence of the joint use of NK cell activation and NKT cell activation with anti-cancer agent, radioactivity and steroid therapy and has found that the thing having a serious influence on CTL activation has no influence in the case of activation of NK cells and NKT cells in the joint use with anti-cancer agent, radioactivity and steroid therapy in the common administering method and dose. As a result, in the therapy of cancer using NK cell activator and NKT cell activator, joint use with anti-cancer agent, radioactivity and steroid therapy is now possible whereby more anti-cancer effect is achieved.

Two kinds of immune systems relate to anti-tumor immunological competence and one of them is (1) a CTL activation system of TNF-α (tumor necrosis factor α) → IFN-γ → IL-12 → killer T cell (CTL) while another is (2) a system of NKT cell activation or a system of NK cell activation. In a novel immunotherapy for cancer (NITC) until now, results of therapy in the same degree are noted in those two systems. Thus, there have been noted examples where therapeutic effect is achieved as a result of activation of the above mentioned system of (1) IL-12 → killer T cell activation → apoptosis and other examples where therapeutic effect is achieved as a result of activation of the above mentioned (2) NKT cell activation system or NK cell activation system.

When joint therapy with anti-cancer agent, radioactivity or steroid is carried out, the system of TNF-α → IFN-γ → IL-12 → killer T cell between the above-mentioned two kinds of immune systems is significantly hindered. It has been newly found that, on the other hand, the NKT cell activation system and the NK cell activation system are not hindered at all.

As a result of new reconstitution of therapeutic method for cancer on the basis of such a phenomenon, another embodiment of the present invention has been achieved. Thus, when the joint therapy with anti-cancer agent, radioactivity or steroid is integrated with cancer therapy, the joint therapy is possible and the therapeutic effect becomes good when the above-mentioned immune system (2) is strong. However, when the above-mentioned immune system (2) is weak and only the immune system (1) is strong, it is presumed that the joint therapy results in failure. In that case, it is necessary to administer α-1,3-sugar (a saccharide substance having an α-1,3-steric structure) which is an activating substance for NKT cells or NK cells or, in other words, to jointly use an α-1,3-sugar which potentiates the above-mentioned immune system of (1). Alternatively, when an anti-cancer agent is administered, it is inevitable to apply a low-concentration chemotherapy which is an administering method which does not hinder the above-mentioned immune system (1) or, in other words, to apply an administration method of low concentration of 5FU, UFT, Mifurol, Furtulon or CDDP (5 µg to 10 µg) or a low-concentration anti-cancer agent such as taxotere, taxol, Adriamycin, mitomycin and CPT-11. Similarly it is necessary to select application of a low-dose irradiation in radiotherapy or to select a low-concentration administration in steroid therapy.

Accordingly, when anti-cancer agent, radioactivity or steroid therapy is carried out, assay of various immunological competences of the subject to whom such a therapy is applied is inevitable. On the basis of the result of the assay, when the above immune system (2) is strong, it is necessary for maintain that to administer a substance having activating ability for NKT cells or NK cells or, in other words, a saccharide substance having an α-1,3-steric structure while, when immune strength of the above system (2) is weak, it is necessary to administer a saccharide substance having an α-1,3-steric structure in large quantities or directly into the body such as, for example, to administer by means of injection. When only immunological competence of the above (1) system acts, it is necessary to administer an anti-cancer agent to an extent of not hindering the above immune system (1), i.e. in a low concentration, or to administer an IL-12 inducing substance in large quantities for potentiating the above immune system (1) so that the above immune system (1) by administration of an anti-cancer agent is quickly started.

The information as mentioned above provides methods for testing, diagnostic and therapeutic methods for cancer, characterized in that, a marker for each cancer action is measured in a sample excised from a patient suffering from cancer and the measured value is analyzed for each tissue and/or tissue type whereupon a formulation is selected for each tissue and/or tissue type of each cancer.

When such information is carried on a medium which utilizes law of nature, a commercial medium is resulted and such a commercial medium provides a useful commercial method.

Examples of the saccharide substance of an α-1,3-steric structure are nigerooligosaccharide (TSO), fucoidan and oligosaccharide sulfate.

Nigerooligosaccharide is a saccharide containing 3-O-α-D-glucopyranosyl-D-glucose as a constituting unit. Representative examples thereof are nigerose, nigerosyl glucose and nigerosyl maltose.

With regard to a commercially available nigerooligosaccharide, a nigerooligosaccharide liquid sugar (sold by Takeda Food products, Ltd.) is exemplified and main nigerooligosaccharides contained therein are (1) nigerose (α-D-Glcp-(1,3)-D-Glc), (2) nigerosyl glucose (α-D-Glcp-(1,3)-α-D-Glcp-(1,4)-D-Glc and (3) nigerosyl maltose (α-D-Glcp-(1,3)-α-D-Glcp-(1,4)-α-D-Glcp-(1,4)-D-Glc (where Glc and p are abbreviations for glucose and pyranose, respectively).

In its narrow sense, fucoidan is a polysaccharide containing fucose sulfate in which one molecule of sulfuric acid is bonded to 2 to 6 molecules of fucose and a fucoidan-like polysaccharide where xylose or uronic acid is contained therein is called "fucoidan" in a level of food. Fucoidan is made into a preparation by, for example, the following manner that sea tangle is disintegrated and made into chips, aqueous solution components are extracted, residue after the extraction is removed by centrifugal separation, low-molecular substances such as iodine and sodium chloride are removed by ultrafiltration and freeze-drying is carried out.

Examples of fucoidan are fucoidan derived from *Kjellmaniae crassifolia* and fucoidan derived from *Cladosiphon okamuranus*. In fucoidan derived from brown algae such as *Kjellmaniae crassifolia*, there are at least three kinds of fucoidans, i.e. F-fucoidan (polymer of α-L-fucose), U-fucoidan (in which β-D-glucuronic acid and α-D-mannose are main chains having α-L-fucose as a side chain) and G-fucoidan (β-D-galactose is a main chain having α-L-fucose as a side chain) and, in all of them, fucose is sulfated.

An example of a sulfated oligosaccharide is an extract derived from *Poryphyra Yezaensis* manufactured by K. K. Shirako. Main components of the extract are an oligosaccharide of galactan sulfate of an α-1,3-bond and an oligosaccharide of galactan sulfate comprising α-1,3-bond and β-1,4-bond.

The CTL activator, NK activator and NKT activator of the present invention are effective for lung cancer (lung squamous carcinoma, lung adenocarcinoma and small-cell lung cancer), thymoma, thyroid cancer, prostatic cancer, renal cancer, bladder cancer, colon cancer, rectum cancer, esophageal cancer, cecum cancer, urinary tract cancer, breast cancer, uterine cervex cancer, brain tumor, cancer of the tongue, pharyngeal cancer, nasal cavity cancer, laryngeal cancer, stomach cancer, hepatic cancer, bile duct cancer, testicular cancer, ovarian cancer, cancer of uterine body, metastatic bone cancer, malignant melanoma, osteosarcoma, malignant lymphoma, plasmacytoma, liposarcoma when an assay method of the present invention is accompanied and its application method is selected although those cancers are not limitative.

The CTL activator (inducer for IL-12 production and inducer for INF-γ production), NK activator and NKT activator are used in formulations in which the activation is able to induce or potentiate and also the activation is able to be maintained using the result of the assay method of the present invention as an indicator. Thus, dose and administering period by which the activation is able to be induced or potentiated or is able to be maintained are selected on the basis of the indicator and used. To be specific, in the case of a compound having an α-1,3-glucan structure which is an NK activator or an NKT activator, the dose is about 1 g to 40 g per day or, preferably, about 5 g to 20 g per day while, in the case of a compound having a β-1,3-glucan structure which is a CTL activator (inducer for IL-12 production and inducer for INF-γ production), it is about 1 g to 10 g per day or, preferably, about 3 g to 6 g per day. The administering period is usually 10 days to 24 months while administering frequency is once to three times a day and administration for consecutive days is preferred. Preferably, the CTL activator, NK activator and NKT activator are ingested orally. It goes without saying that parenteral ingestion (including intravenous or intramuscular administration) is possible by reducing the dose and preparing into such a quality that they are able to be for parenteral administration.

Method for the assay of cells and each marker will be exemplified as follows.

### (Assay of NKT cells) (Assay of NK cells) (Assay of CD 8)

Assay of NKT cells having NKR-P1 is able to be carried out by assay of cell-surface antigens (CD 3 and CD 161) which are specifically present on the cell surface of NKT cells. To be specific, for lymphocytes in peripheral blood, cells where CD 3 is positive and CD 161 is positive (CD3+CD161+) are tested. Thus, CD 3 and CD 161 which are cell-surface antigens of NKT cells are assayed by means of a two-color test using flow cytometry where monoclonal antibody is used. Here, the expression NKT cells are activated means that, the rate of (CD3+CD161+) NKT cells in the lymphocytes is not less than 10% or, more preferably, not less than 16%. The term reading the NKT cell activating ability means a function by which rate of the NKT cells is increased to not less than 10% or, more preferably, not less than 16% or a function by which rate of the NKT cells before administration of a substance is further potentiated.

Similarly, (CD3-CD161+) means to test the cells where CD 3 is negative and CD 161 is positive. The method has been confirmed to be useful for the assay of NK cells.

Further, CD8+ means to test the cells where CD 8 is positive. The method has been confirmed to be useful for the assay of CTL cells.

In Examples, blood of patients suffering from cancer was used and, with regard to cells in the blood, they are classified in view of being positive and negative for CD 3, CD 161 and CD 8 which are cell-surface antigens and rate of each cell was assayed according to a conventional method by means of a two color test using flow cytometry. At that time, with regard to each of the monoclonal antibodies to CD 3, CD 161 and CD 8, that which was manufactured by Coulter or Becton Dickinson was used.

### (Assay of perforin-producing cells)

With regard to lymphocytes in peripheral blood, assay is carried out according to a common method by a three color test using a flow cytometry for perforin and two of CD 3, CD 8 and CD 161 which are cell-surface antigens. To be specific, a fixing solution is added to the collected blood to fix the cells, a solution permeating the membrane is added, then reaction is carried out after addition of an anti-perforin antibody (manufactured by Pharmingen), further reaction is carried out after addition of PRE-Cy5 labeled secondary antibody (manufactured by Dako), then reaction is carried out after addition of anti-CD3-PE (Coulter 6604627) antibody and anti-CD161-FITC (B-D) antibody and an assay is conducted by means of a flow cytometry. The fact that the perforin production is significant means NK cells or NKT cells are activated. In the drawings, it is abbreviated as PERF.

### (Known assay method for NK cell activity)

The known method is a chromium release method (in which effecter cells (NK cells) are added to target cells (K-562) labeled with ⁵¹Cr followed by incubating and ⁵¹Cr liberated by target cell hindrance is assayed to calculate the activity value) (*Med. Technol*. 21(7), 574-580(1993)).

### (Preparation of a sample for the assay of cytokine)

Firstly, a mononucleosis fraction is separated and prepared from blood. Heparin-added peripheral blood is diluted to two-fold with a phosphate-buffered saline (PBS), mixed, layered on a Ficoll-Conray liquid (specific gravity: 1.077) and centrifuged at 400G for 20 minutes to collect a mononucleosis fraction. After washing, an RPMI-1640 medium to which 10% fetal bovine serum (FBS) were added was added thereto so as to make cell numbers 1 × 10⁶. Phytohemagglutinin (manufactured by Difco) was added to 200 µl of the resulting cell suspension to make its concentration 20 µg/ml and incubated at 37°C for 24 hours in a 96-well microplate in the presence of 5% CO₂ to give a sample for assay of cytokine in the incubated cell solution.

### (Assay of IL-12)

In an assay of the amount of IL-12, it is possible to utilize clinical and biochemical tests which have been known *per se* and an assay kit in accordance with an enzyme-linked immunosorbent assay (ELISA) which is able to be available from R&D Systems or MBL is used. Here, an assay kit of R&D Systems was used. Actually, 50 µl of Assay Diluent RD1F (a diluted solution for the assay) and 200 µl of a standard solution or the sample prepared in Example 1 were placed in each well of the 96-well microplate and made to react for 2 hours by allowing to stand at room temperature. After that, each 200 µl of anti-IL-12 antibody labeled with horse raddish peroxide (HRP) were placed and allowed to stand at room temperature for 2 hours. The reaction solution in each well was taken out, washed for three times, each 200 µl of a coloring substrate solution were placed and allowed to stand at room temperature for 20 minutes and each 50 µl of a solution for stopping the enzymatic reaction were placed. Absorbance of each well at 450 nm was measured by Emax (manufactured by Wako Pure Chemical) using 550 nm as a control. Amount of IL-12 is expressed as pg/ml. Here, the inducing ability for IL-12 production means a function where the amount of IL-12 produced by stimulation of the mononucleosis fraction in peripheral blood is potentiated to an extent of not less than 7. 8 pg/ml or a function where potentiation is resulted from the production amount of IL-12 before administration of a substance.

### (Assay of IFN-γ)

Assay of IFN-γ was carried out by means of enzyme immunoassay (EIA) using an IFN-γ EASIA kit of BioSource Europe S. Actually, each 50 µl of a standard solution or the above-prepared sample diluted to two-fold were placed and each 50 µl of an HRP-labeled anti-IFN-γ antibody were placed to each well of a 96-well microplate followed by being made to react at room temperature for 2 hours with shaking. The reaction solution of each well was removed and washed for three times, each 200 µl of a coloration substrate solution were placed and made to react at room temperature for 15 minutes with shaking and each 50 µl of a stopping solution for enzymatic reaction were placed. Absorbances of each well at 450 nm and 490 nm were measured by Emax (manufactured by Wako Pure Chemical) using 630 nm as a control. Amount of IFN-γ is expressed as IU/ml.

### Examples

The present invention will be specifically illustrated by way of the following Examples although the present invention is not limited to those Examples only.

### Example 1

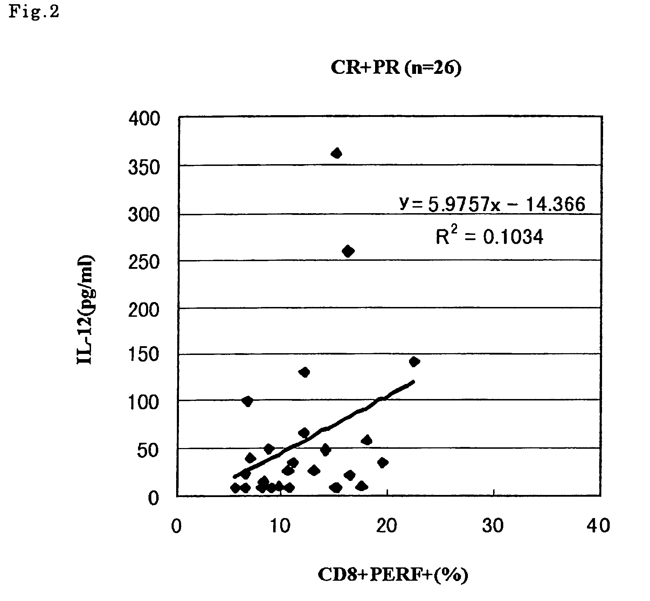

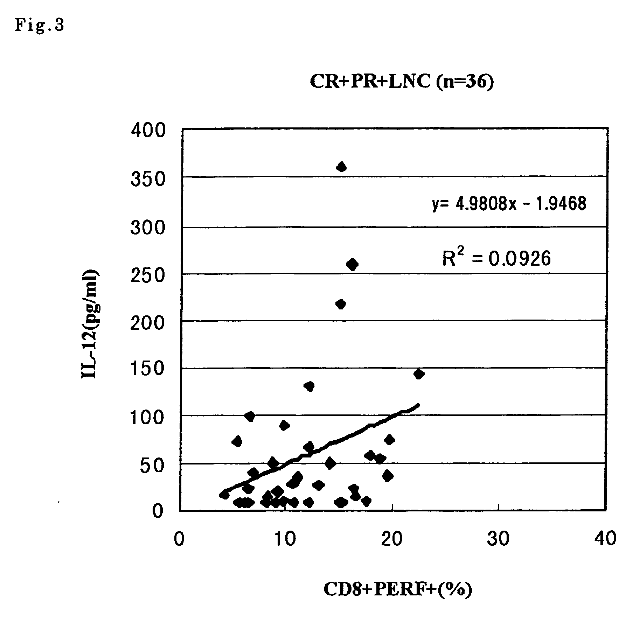

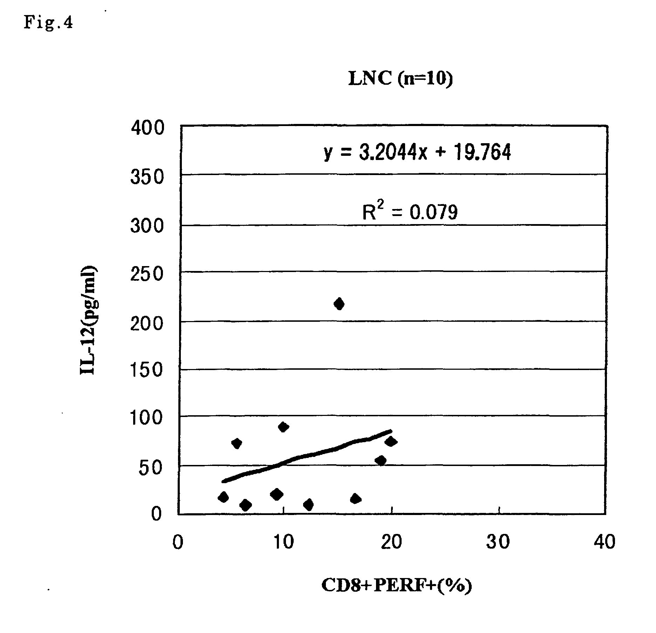

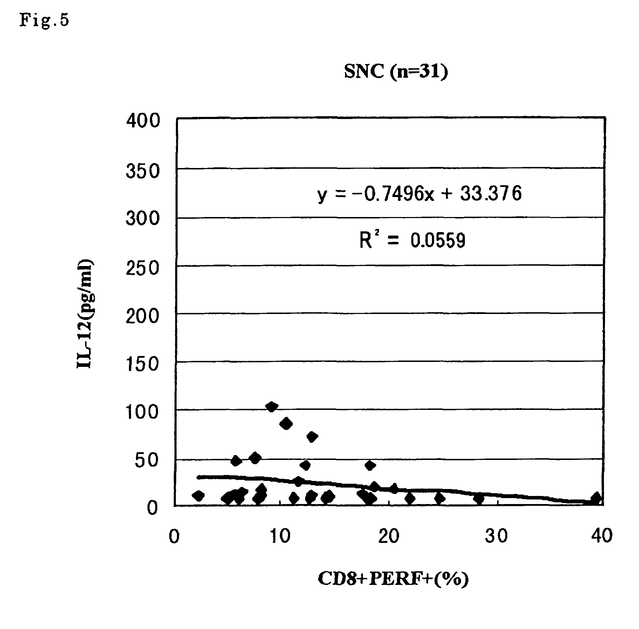

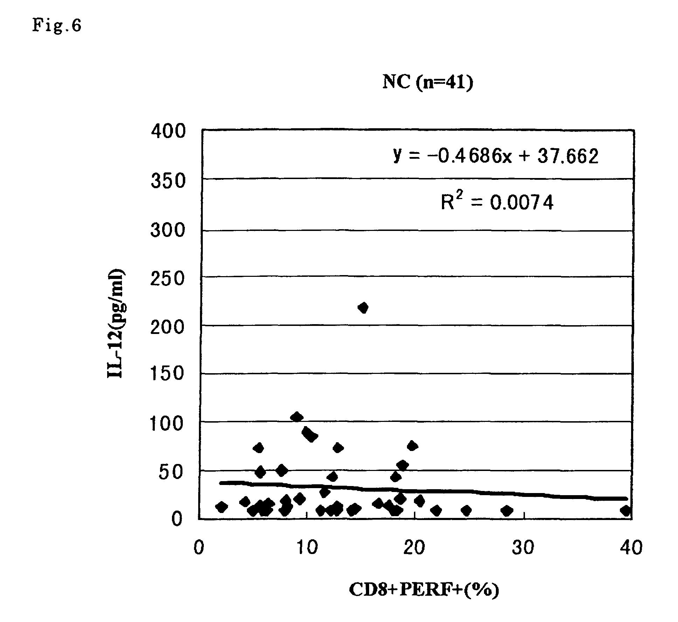

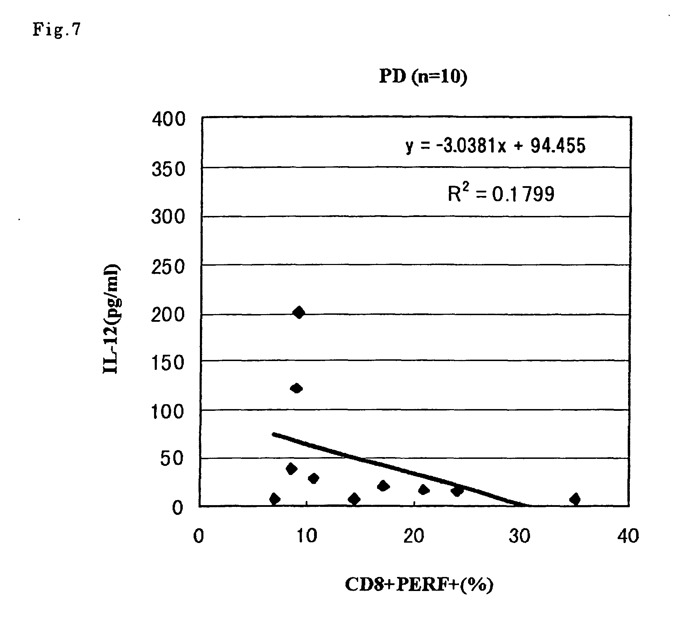

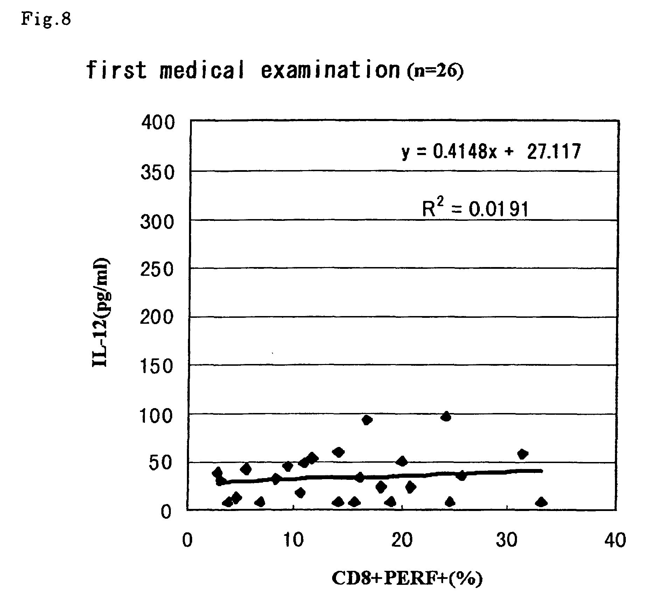

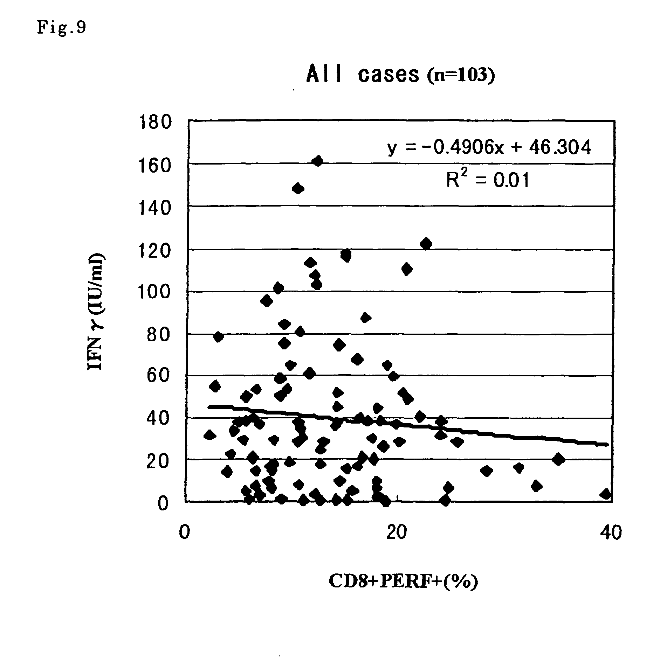

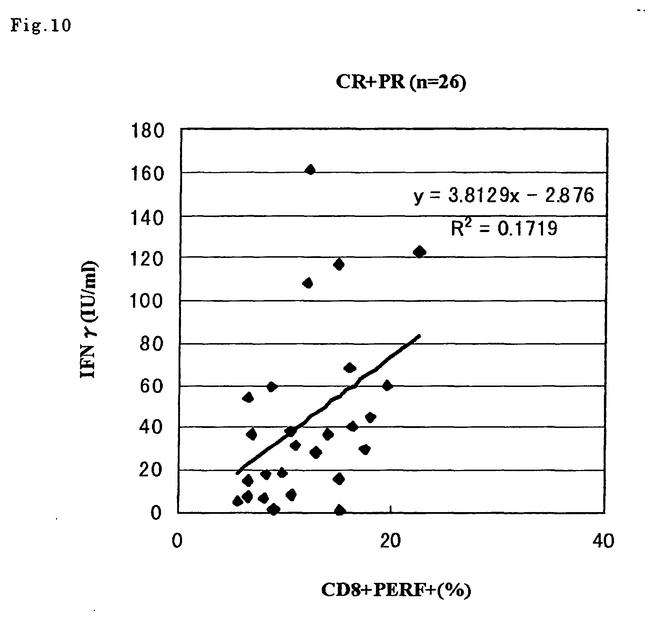

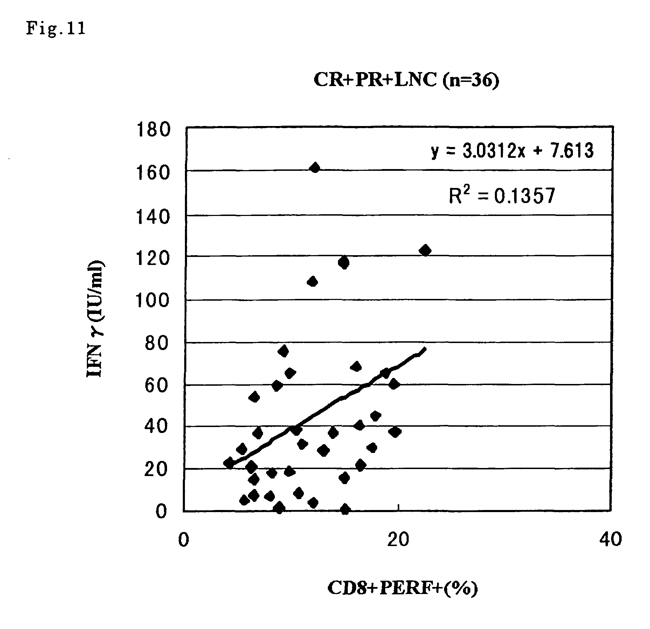

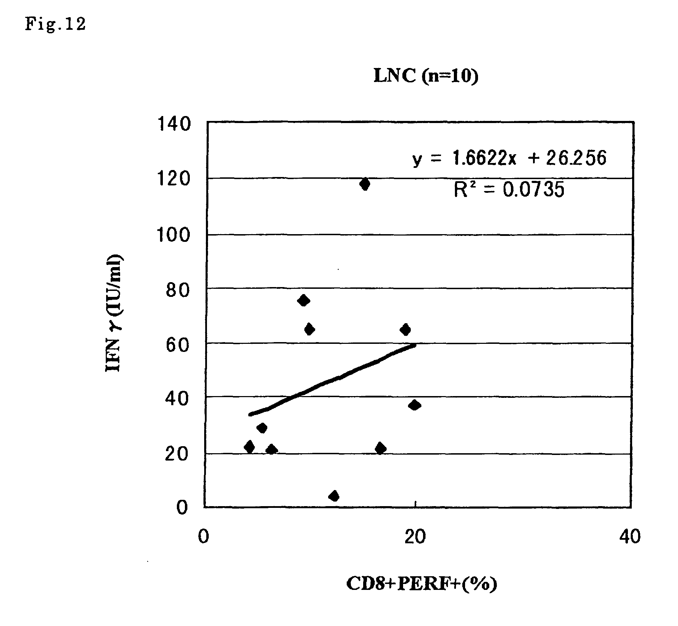

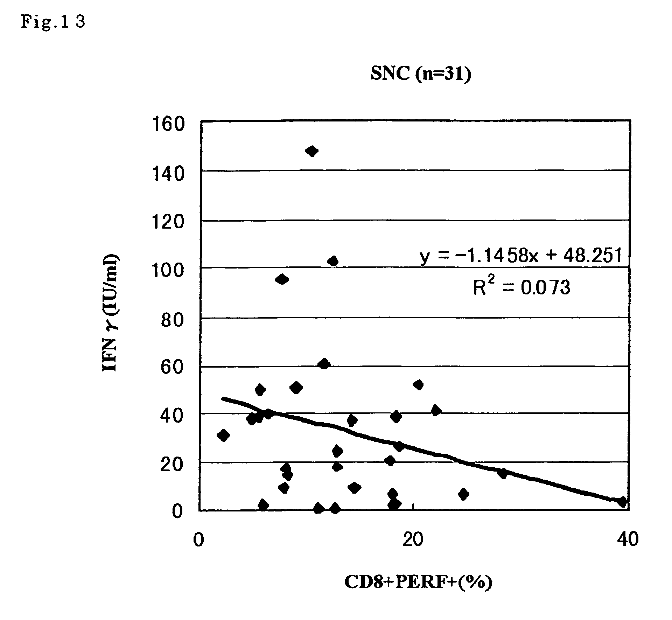

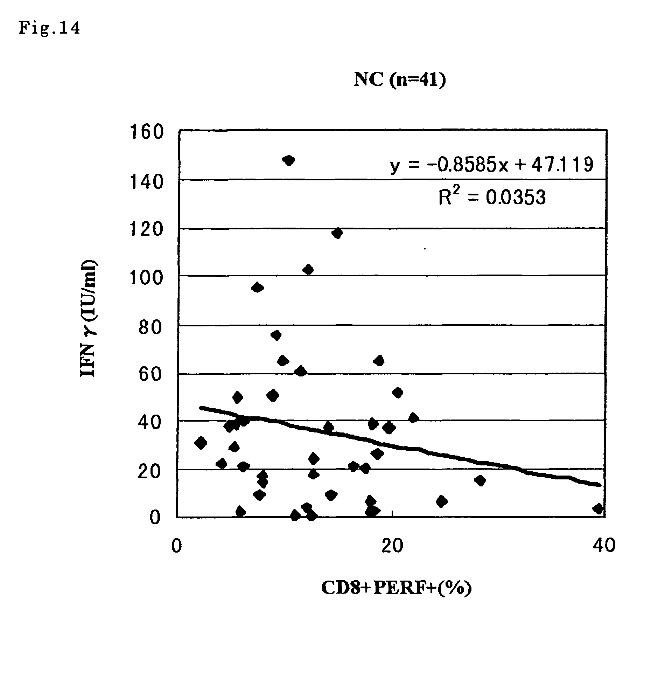

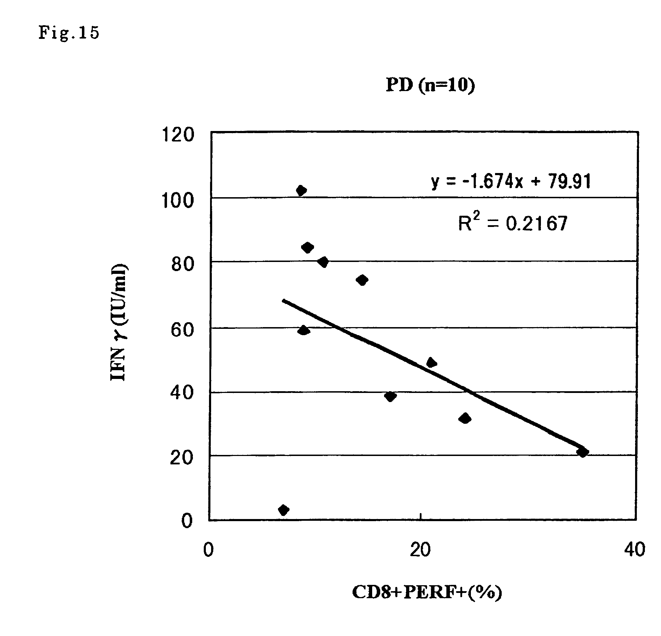

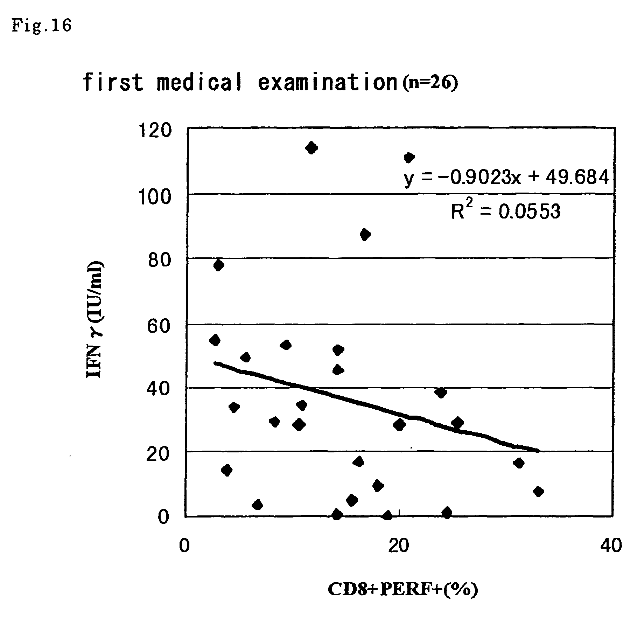

ILX (K. K. Tozai Iyaku Kenkyusho), cartilage of shark (Seishin Kigyo) and a saccharide having an α-1,3-structure (nigerooligosaccharide) were administered to a patient according to a recommended formulation for each of them. In the drawings, CR, PR, LNC, SNC, NC and PD were used in the meanings of usual classifications in cancer therapy. Figs. 1 to 8 show the relation between the induced amount for IL-12 production (pg/ml) (ordinate) and cell rate (%) of CD8(+)perforin(+) (abscissa) in 103 cases. In all of Fig. 1, SNC of Fig. 5, NC of Fig. 6, PD of Fig. 7 and first-time patients of Fig. 8, there was no correlation at all while, in the effective cases in the therapy of Fig. 2 (CR + PR) , Fig. 3 (CR + PR + LNC) and Fig. 4 (LNC), correlation was confirmed between both. Figs. 9 to 16 show the relation between the induced amount of INF-γ production (IU/ml) (ordinate) and the cell rate (%) of CD8(+)perforin(+) (abscissa) in 103 cases. In all of Fig. 9, SNC of Fig. 13, NC of Fig. 14, PD of Fig. 15 and first-time patients of Fig. 16, there was no correlation at all while, in the effective cases in the therapy of Fig. 10 (CR + PR), Fig. 11 (CR + PR + LNC) and Fig. 12 (LNC), correlation was confirmed between both. As a result, it has been proved that the induction of IL-12 production and the induction of INF-γ production are in the same meanings as the assay system of CD8(+)perforin(+) and accordingly that the assay of CD8(+)perforin(+) means the assay of CTL activation.

### Example 2

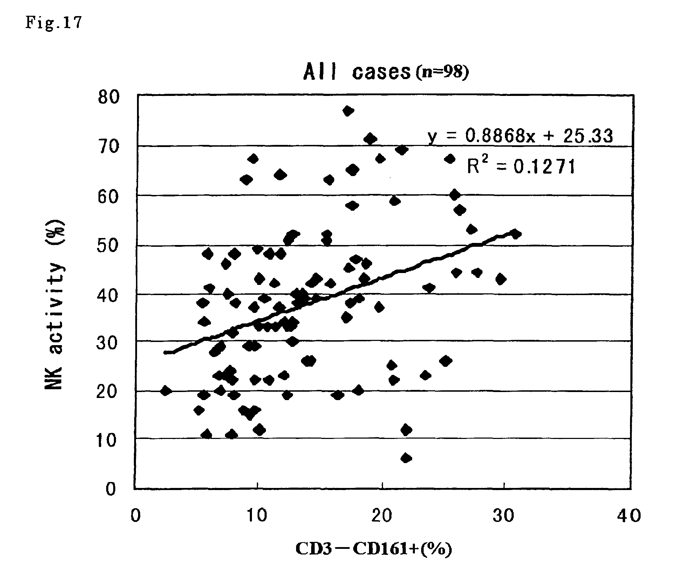

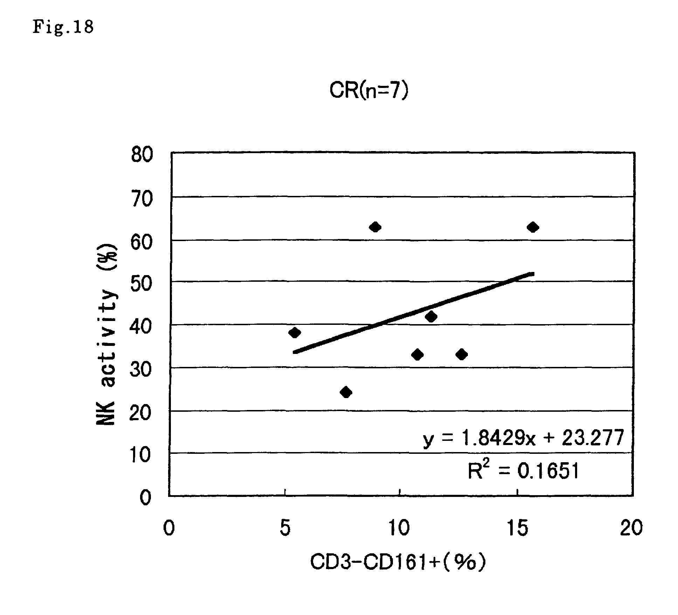

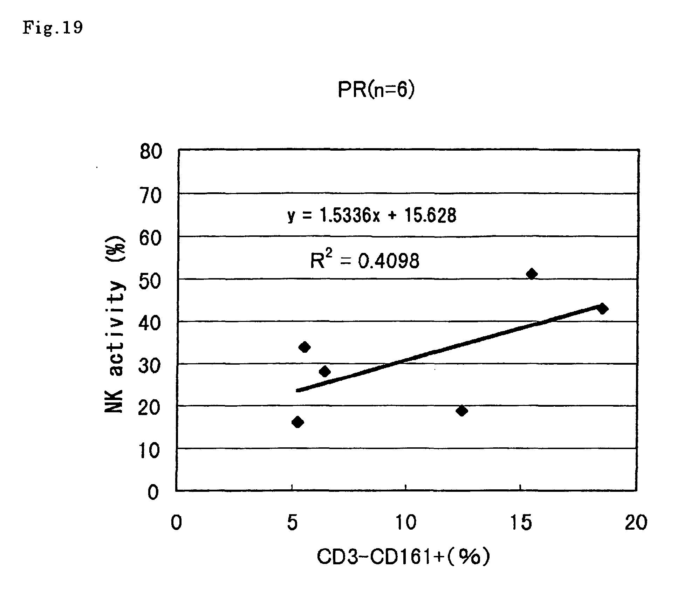

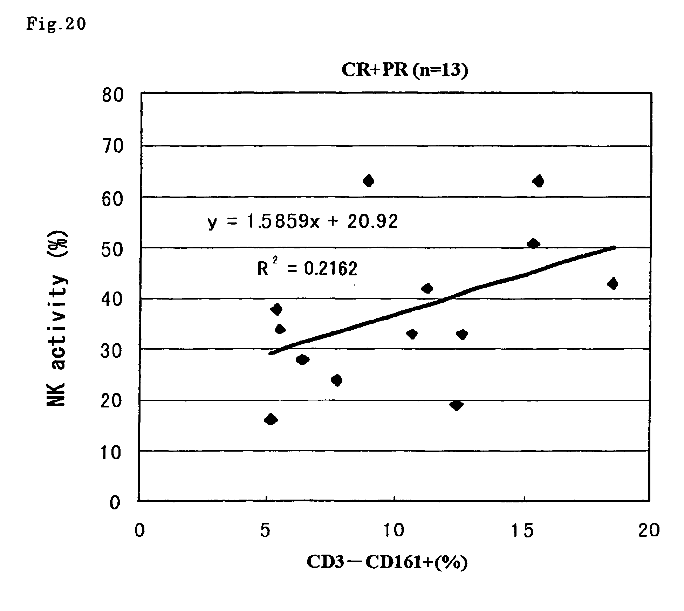

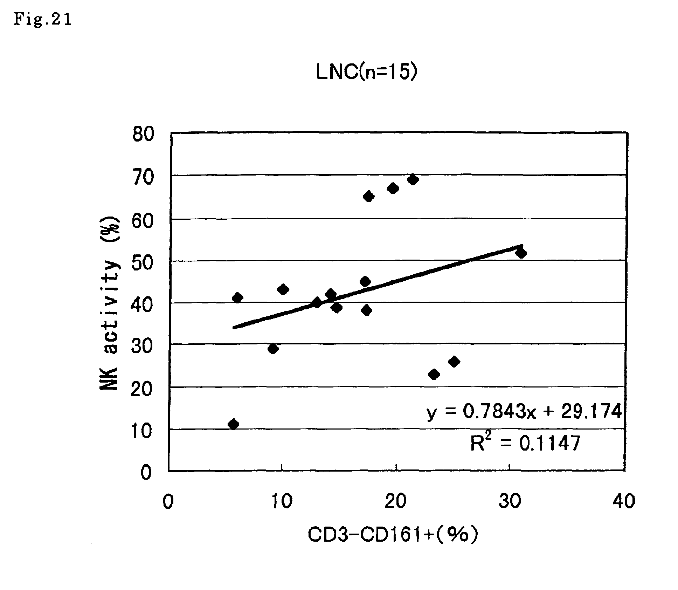

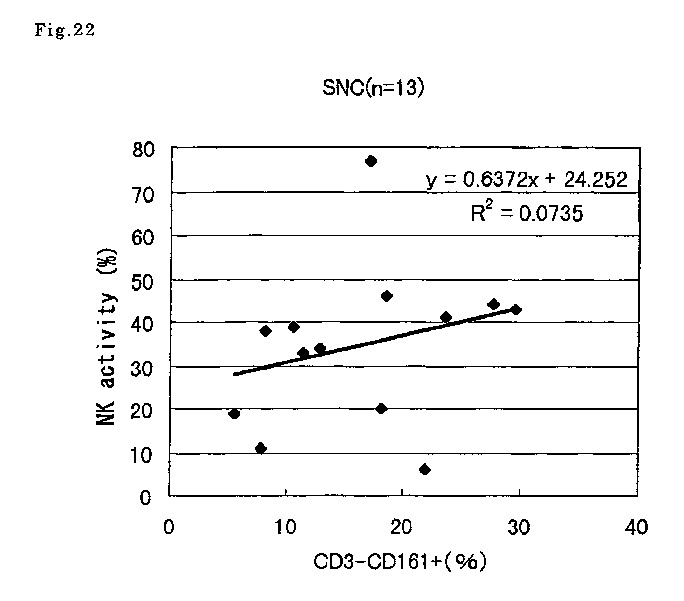

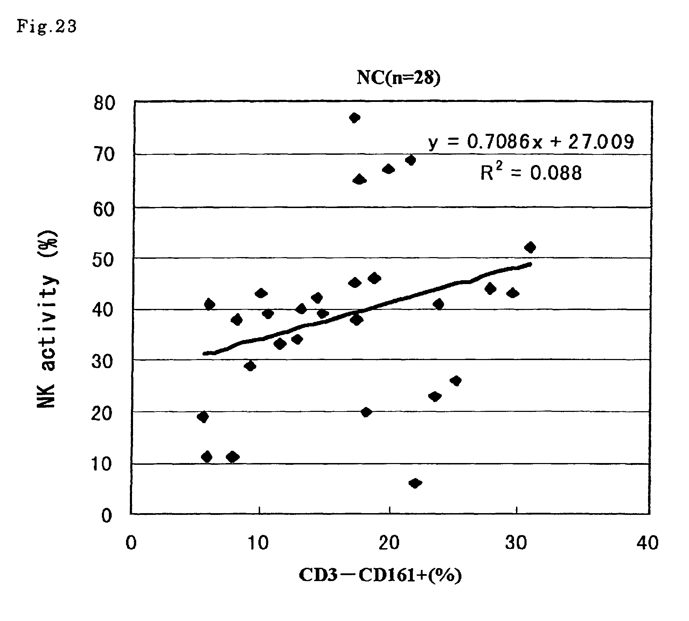

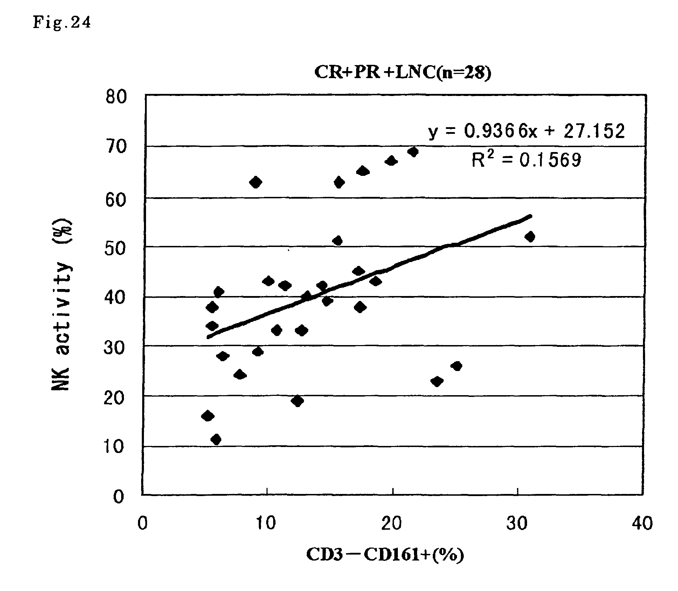

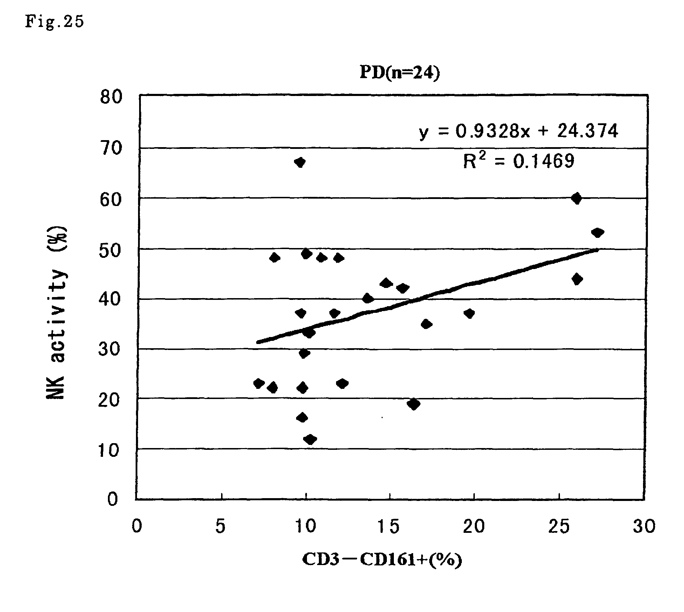

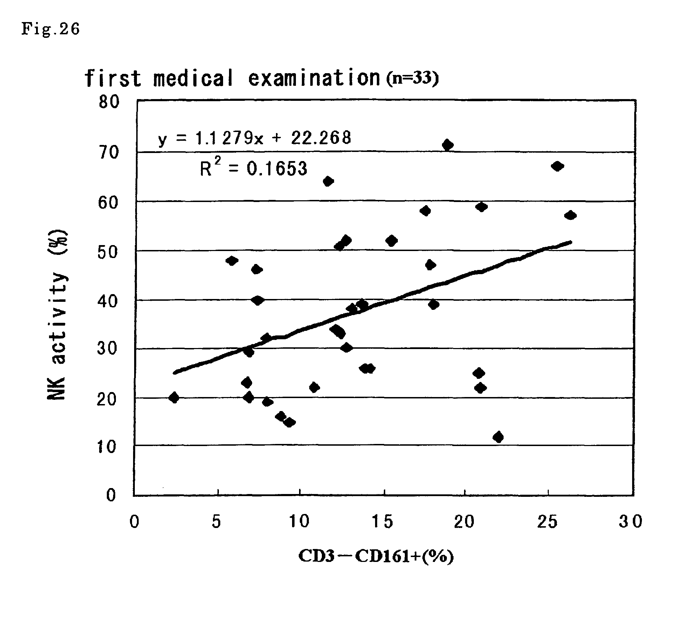

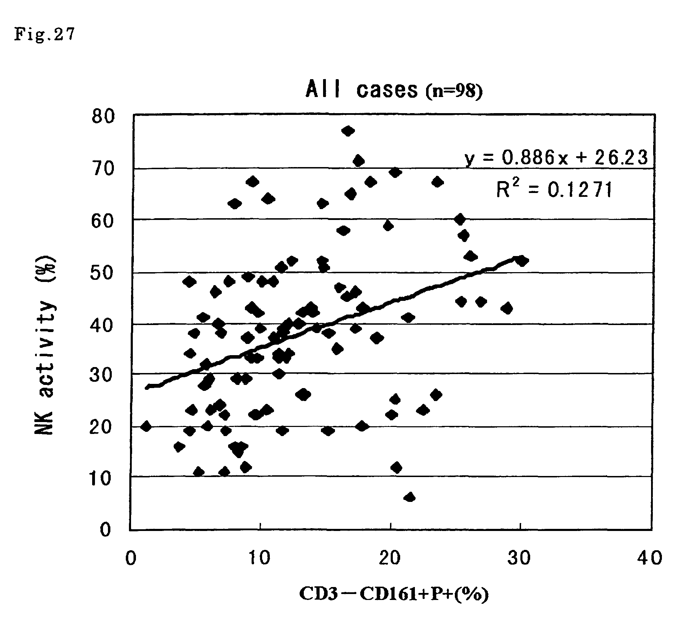

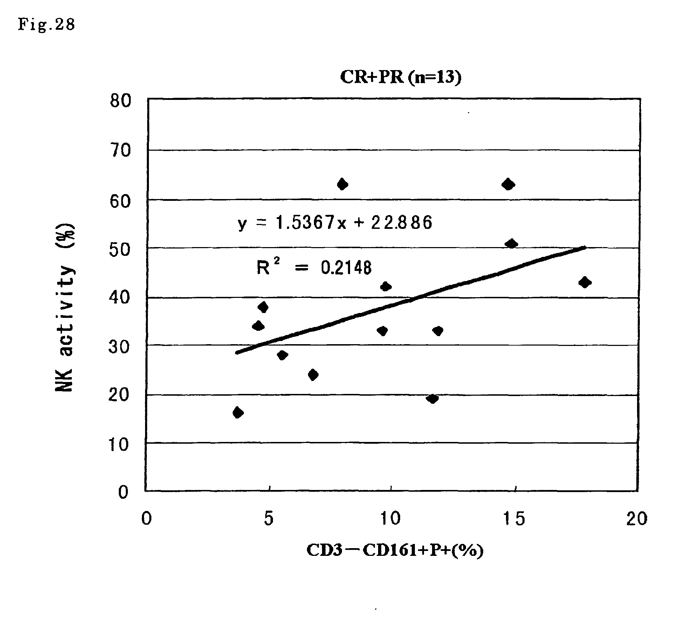

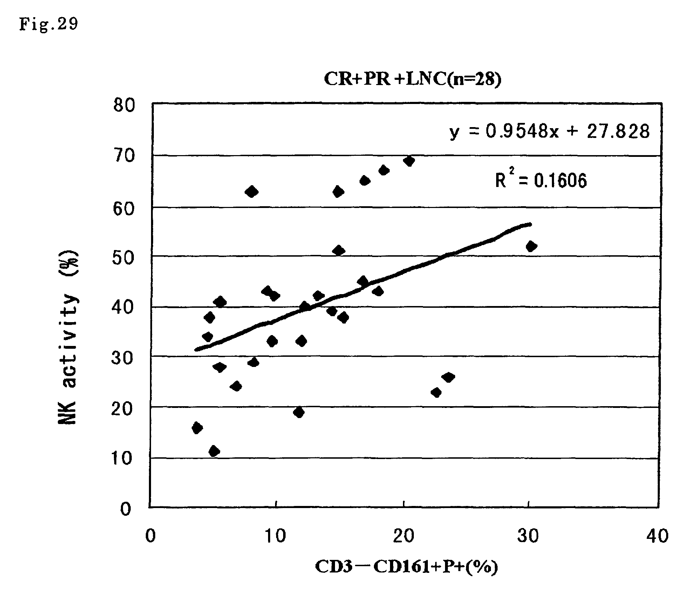

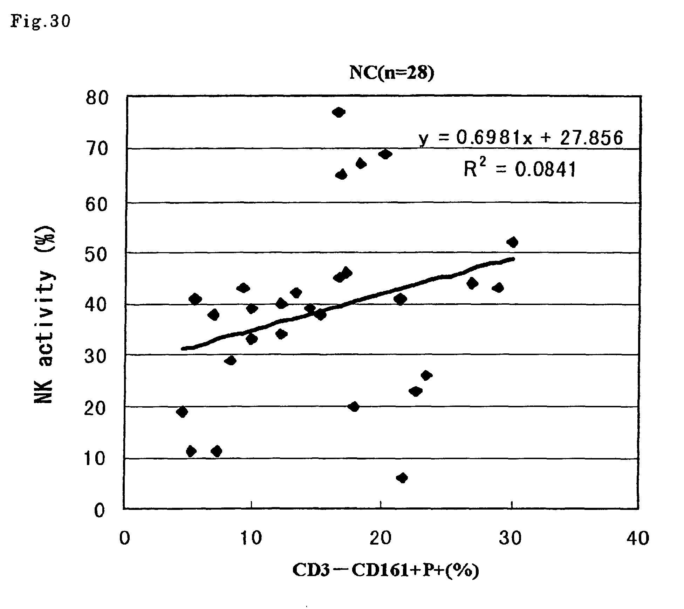

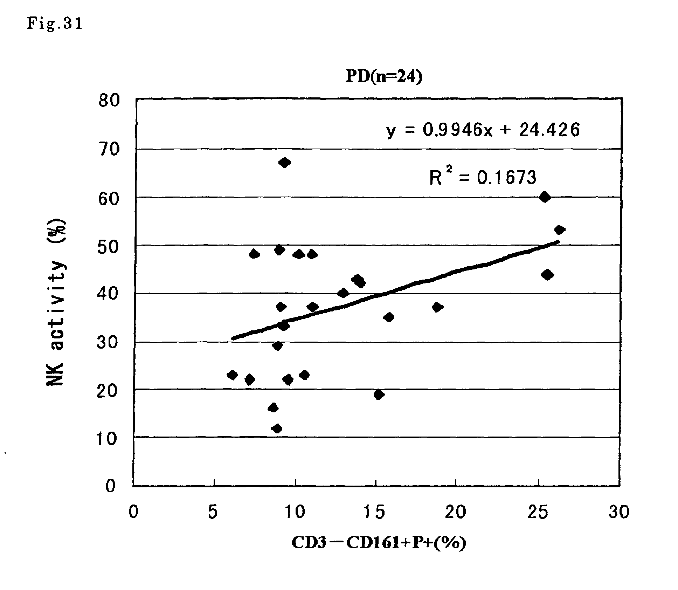

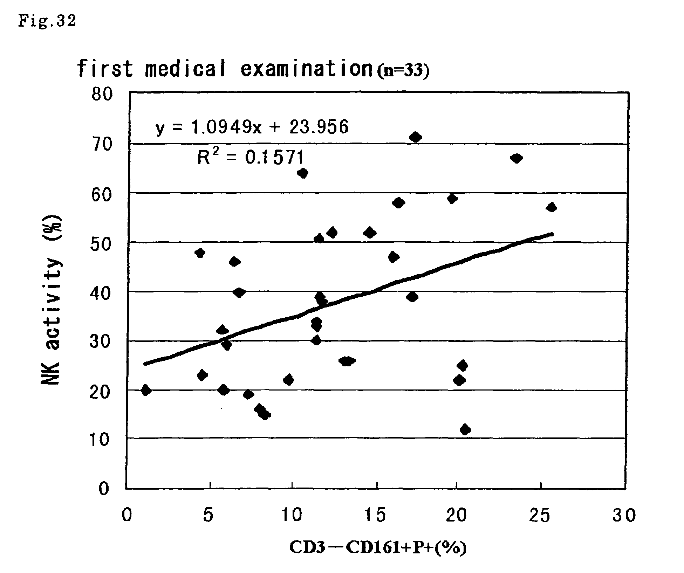

In the same treated group of patients (98 cases) as in Example 1, significance of the assay method for NK activity (the conventional method) and the assay of CD3(-)CD161(+) of the present invention was investigated. Figs. 17 to 26 show the relation between the NK cell activity (%) and CD3(-)CD161(+) (%) and Figs. 27 to 32 show the relation between then NK activity (%) and CD3(-)CD161(+)P(+) [where P(+) means production of perforin]. As a result, it has been proved that the NK activity assay by the conventional method has the same meaning as the assay system of CD3(-)CD161(+) and CD3(-)CD161(+)perforin(+) and accordingly that the assay of CD3 (-) CD161 (+) means the assay of NK activity.

### Example 3

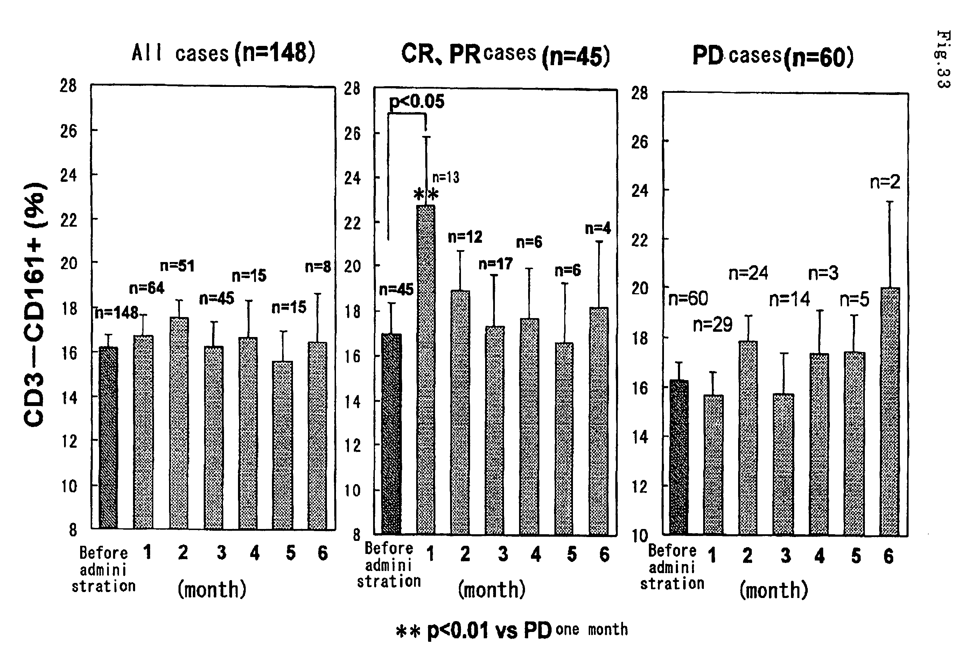

In the same treated group of patients (148 cases) as in Example 1, particularly, in the cases to which nigerooligosaccharide (TOG) was administered as an α-1,3-structure compound in a recommended treatment, CD3(-)CD161(+) was assayed and comparison before starting the administration of TOG with after that and comparison of effective cases with ineffective cases were carried out. Fig. 33 shows that, in effective cases for CR + PR cases, the CD3(-)CD161(+) shows high values. Thus, it has been found that the saccharide of an α-1,3-structure achieves the activation of NK cells.

### Example 4

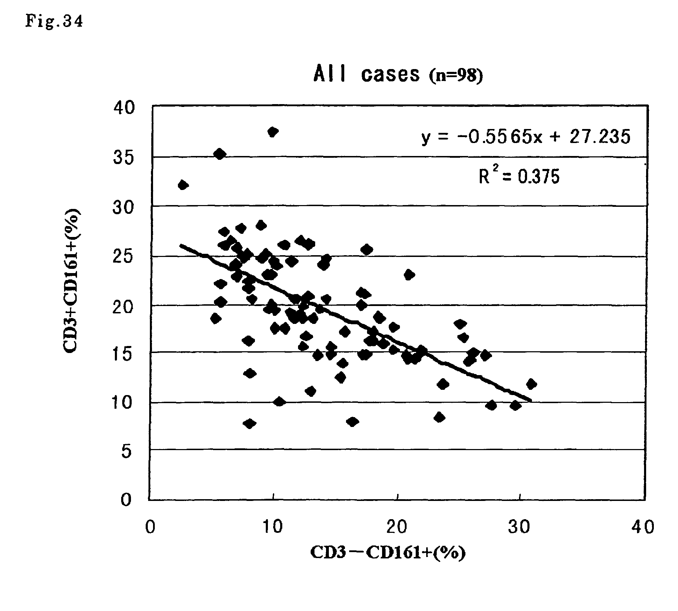

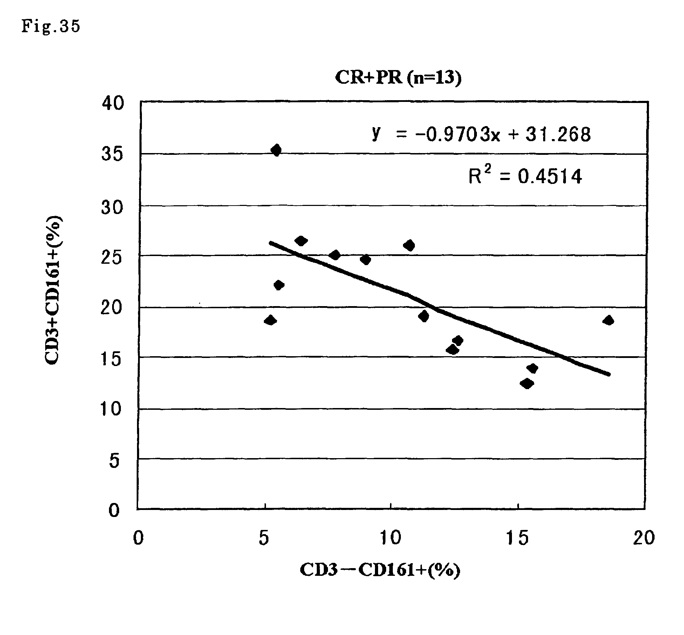

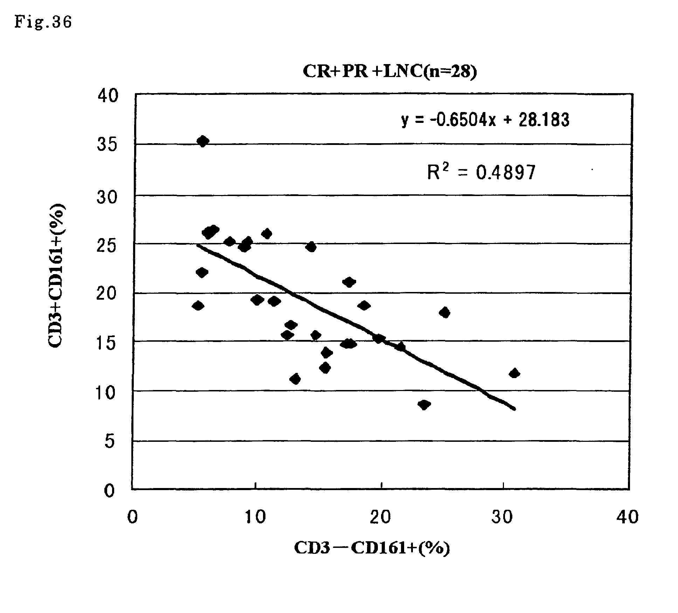

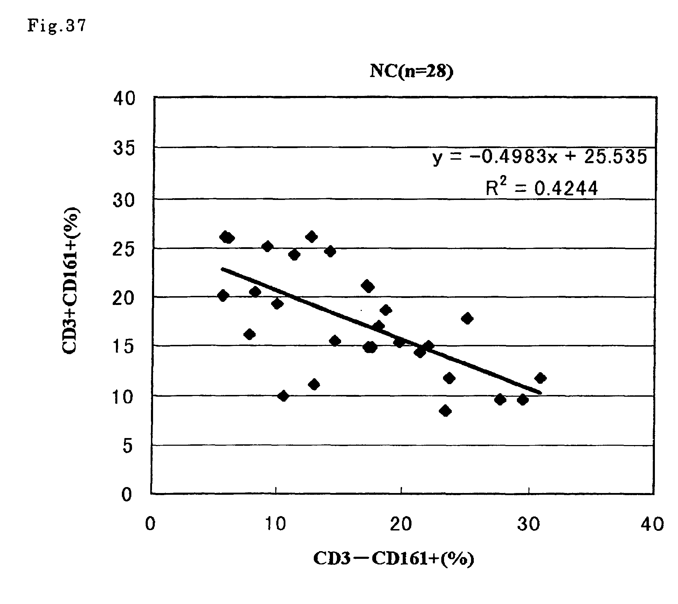

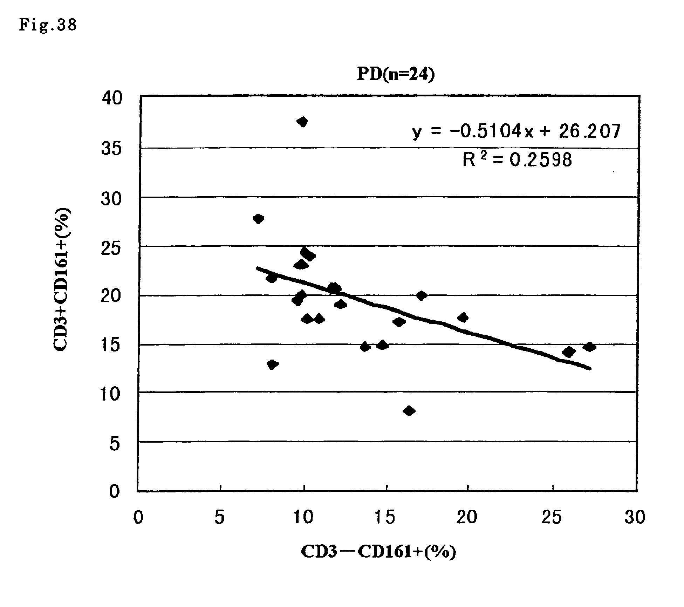

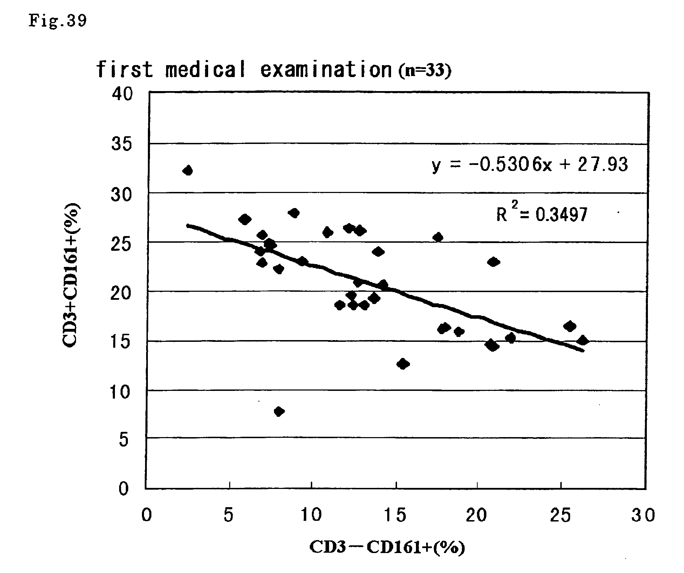

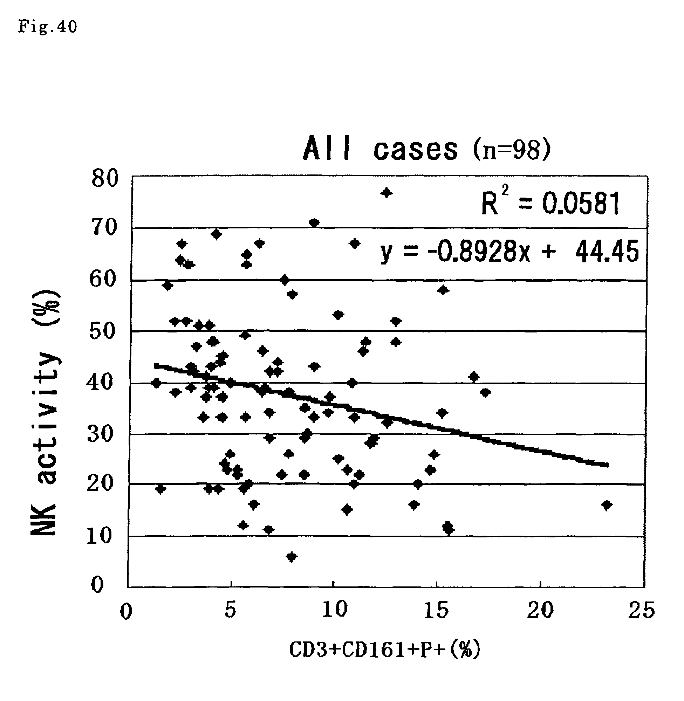

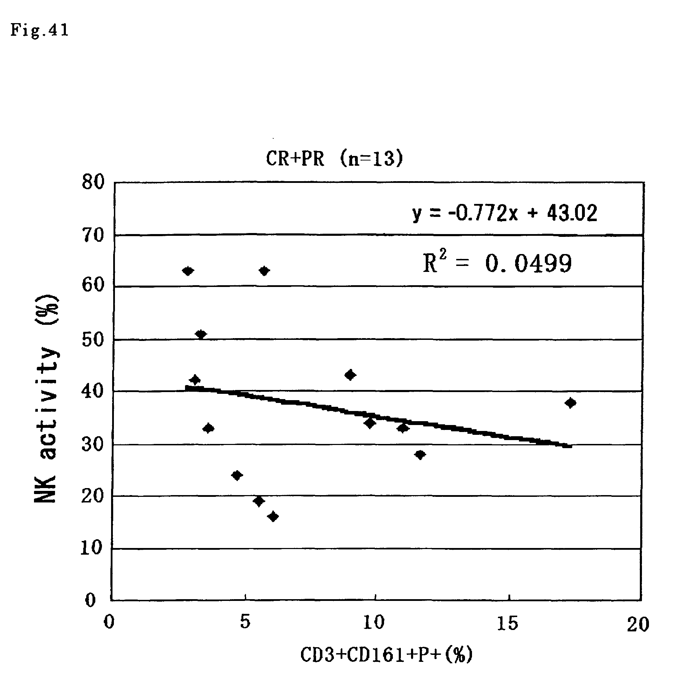

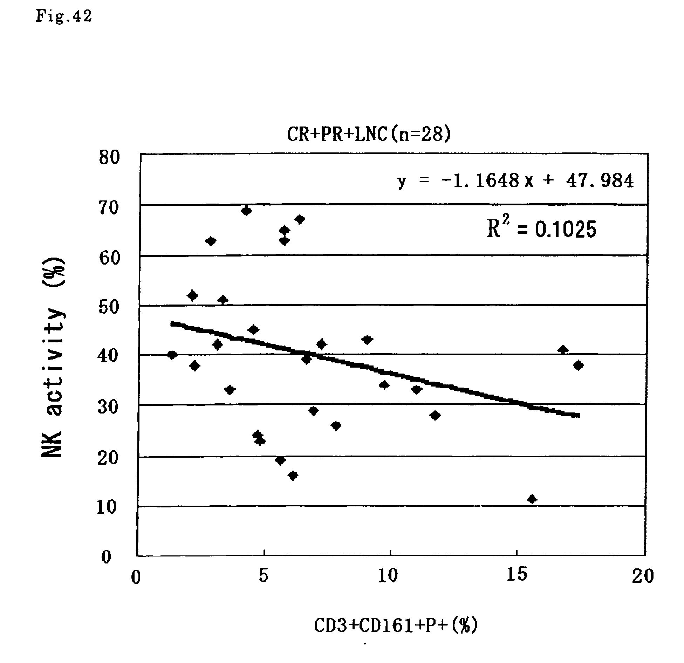

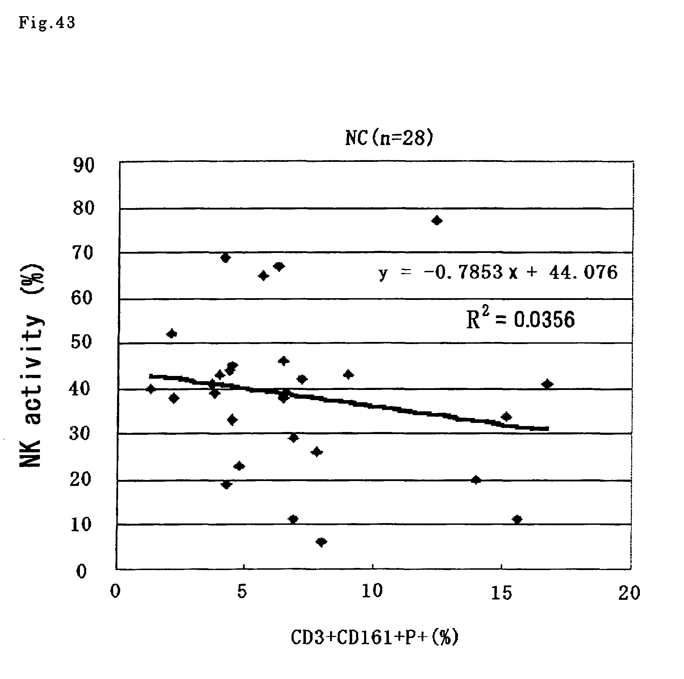

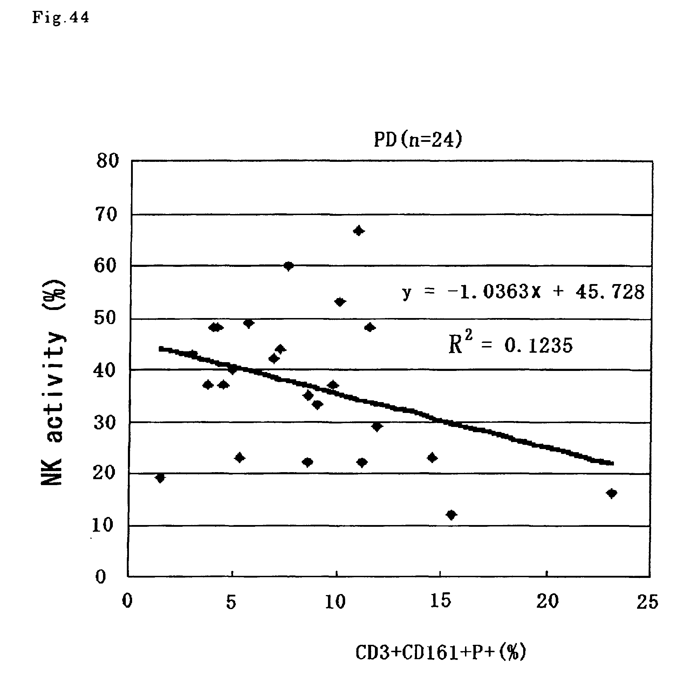

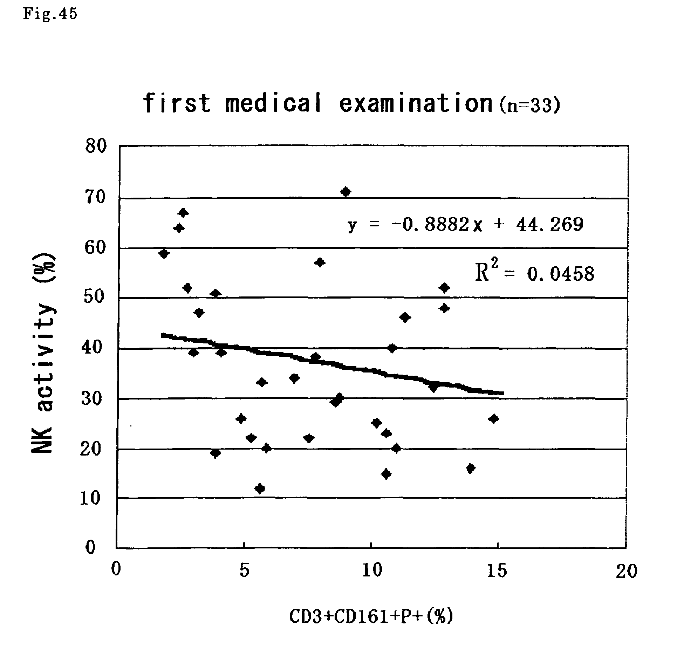

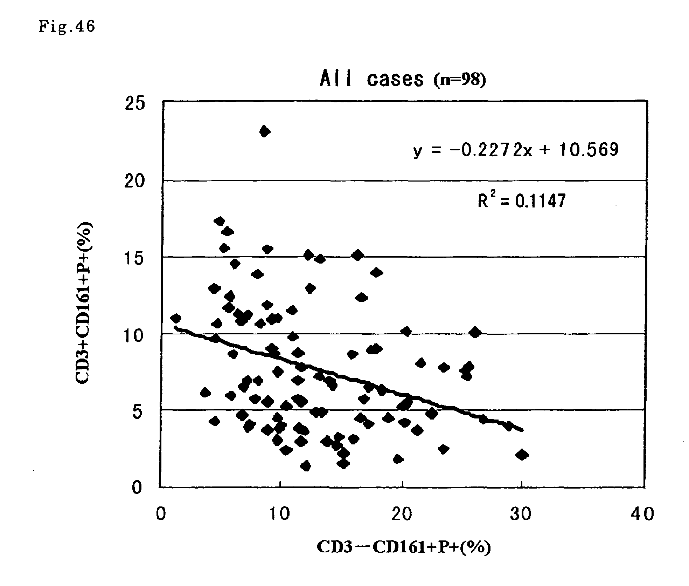

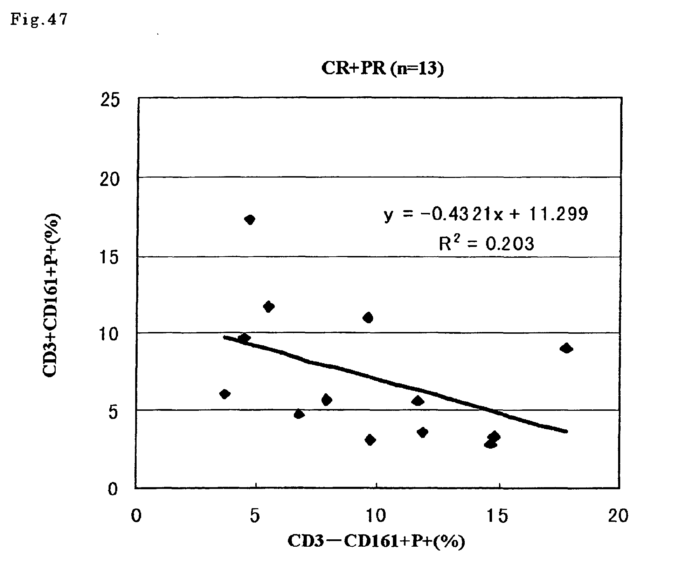

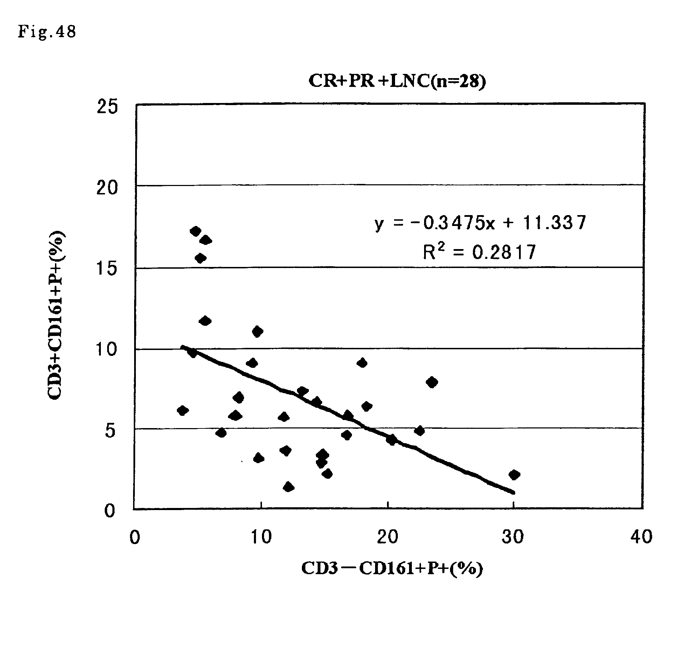

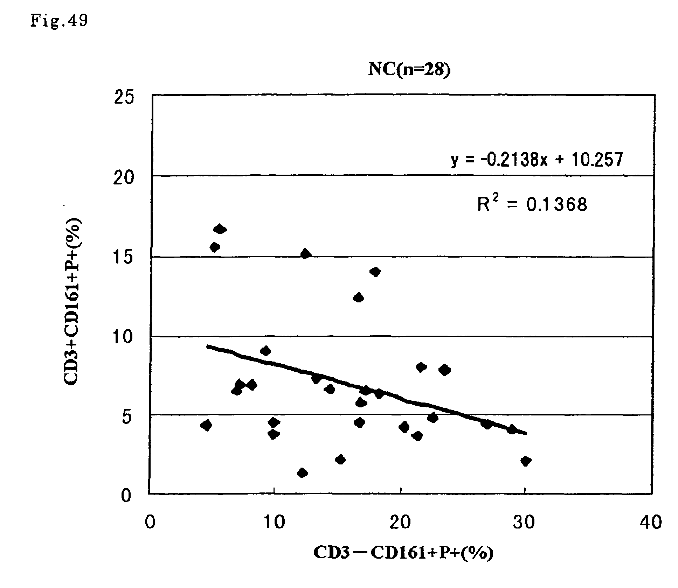

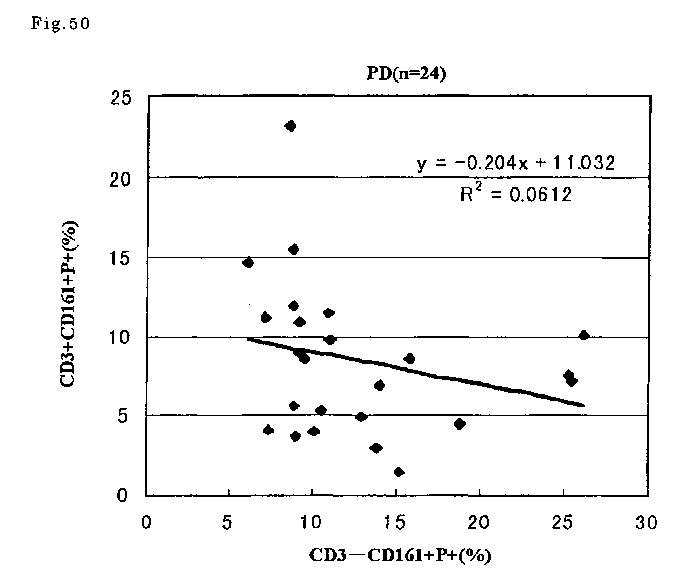

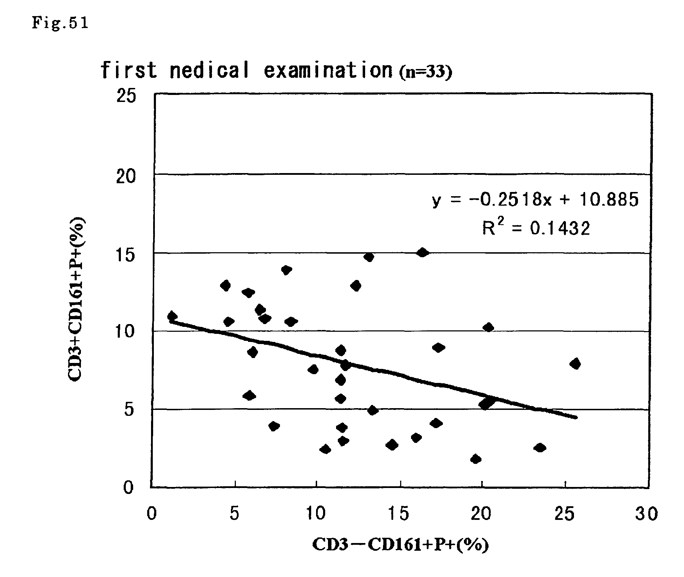

In the same treated group of patients (98 cases) as in Example 1, significance of NK cell activity [either by the conventional method or by the assay of CD3(-)CD161(+) according to the present invention] and NKT cell activity [CD3(+)CD161(+)] was investigated. Figs. 34 to 39 show the relation between CD3(-)CD161(+) [abscissa: NK cell activity] (%) and CD3(+)CD161(+) [ordinate: NKT cell activity] (%) and all of them show an inverse correlation. Figs. 40 to 45 show the relation between the conventional method [ordinate: NK cell activity] (%) and CD3(+)CD161(+)P(+) [in which P(+) means production of perforin] [abscissa: activated NKT cells] and all of them show an inverse correlation. Figs. 46 to 51 show the relation between CD3(-)CD161(+)P(+) [in which P(+) means production of perforin] [abscissa: activated NK cells] and CD3(+)CD161(+)P(+) [abscissa: activated NKT cells] and all of them show an inverse correlation.

### Example 5

In the same treated group of patients (148 cases) as in Example 1, the relation between the cancer type and the contribution of NKT cells or NK cells was analyzed by means of determination of partial regression coefficient by multivariate analysis. As a result, in the case of prostate cancer, the partial regression coefficient of NKT cells [CD3(+)CD161(+)] showed a minus tendency while that of NK cells [CD3(-)CD161(+)] showed a plus tendency. That suggested that, in prostate cancer, contribution of the NK cells is high and an attempt of activation thereof using NK cells as an indicator is an effective therapeutic method for cancer. Similarly, in lung cancer, the partial regression coefficient of NK cells [CD3(+)CD161(+)] is in a plus tendency and that suggested that an attempt of activation thereof using NKT cells as an indicator is an effective therapeutic method for cancer.

### Example 6

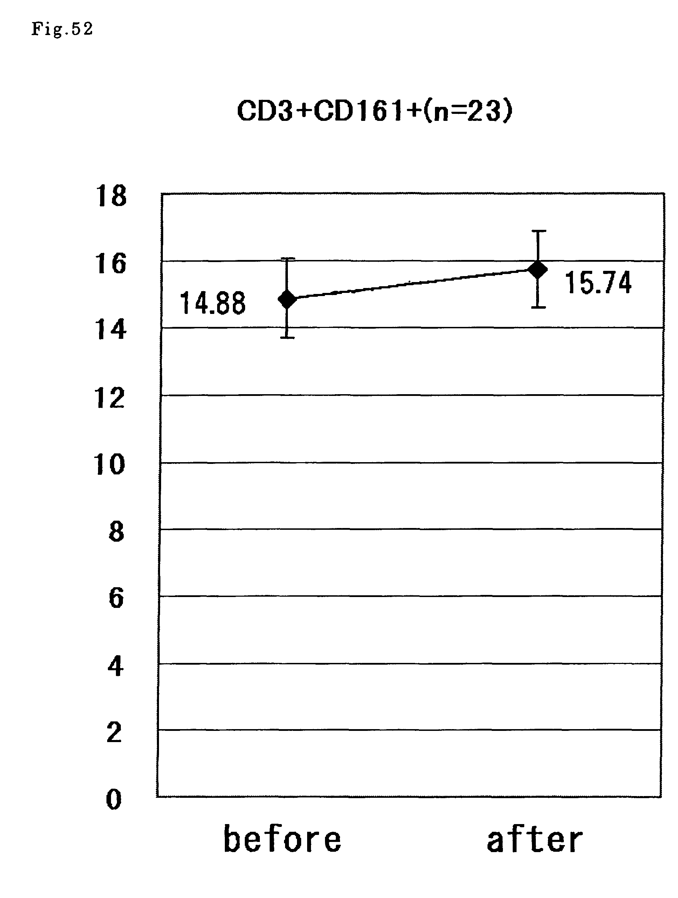

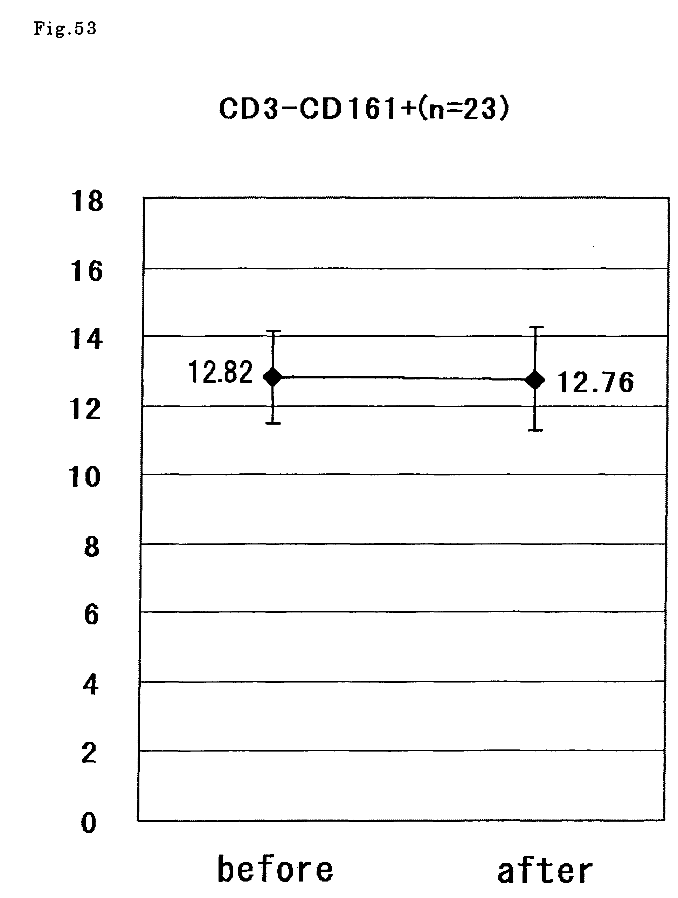

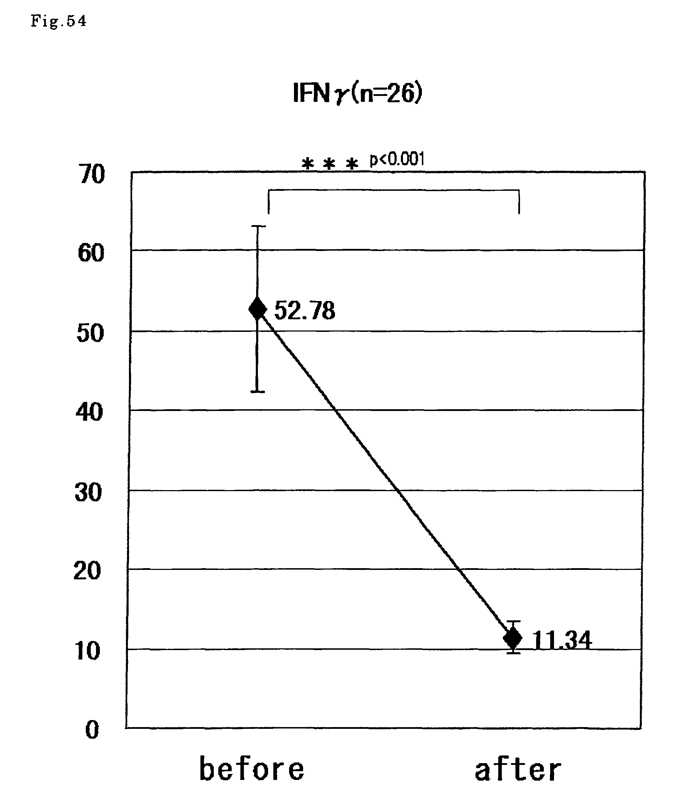

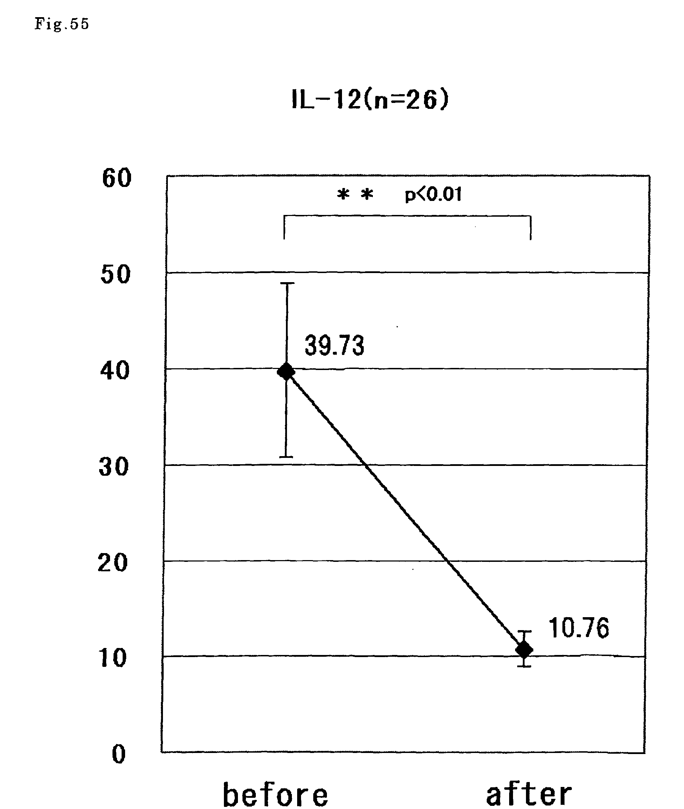

In the same treated group of patients (26 cases) as in Example 1, each indicator after addition (about one month later) of therapy with steroid (6 cases), therapy with radioactivity (7 cases) and therapy with anti-cancer agent (13 cases), NK cells [CD3(-)CD161(+)], NKT cells [CD3(+)CD161(+)], induced production amount of INF-γ and induced production amount of IL-12 were assayed and it was confirmed that, in any of the cases, there was no influence on NK cells [CD3(-)CD161(+)] and NKT cells [CD3(+)CD161(+)] and that significant suppressing effects worked on induced production amount of INF-γ and induced production amount of IL-12 (Figs. 52 to 55).

### Industrial Applicability

As a result of the above experiments and Examples, it has been found that an assay method for activation of NK cells according to the present invention is able to give more correct result than the conventional chromium release method, that a novel assay method for CTL activity is provided, that NK cells are able to be activated by α-1,3-glucan, that activation of NK cells and activation of NKT cells are in an inverse correlation and each of them has a significance as an anti-cancer system, that there is a specificity between the type of cancer and the activation of NK cells and NKT cells and that the activation of NK cells and that of NKT cells are not affected by anti-cancer agent, radioactivity and steroid therapy whereupon an epoch-making result in the therapy of cancer has been achieved.

## Claims

1. A method for an assay of the immune function of human being in a novel immunotherapy where at least one of the following assays is carried out for lymphocytes in peripheral blood:
1) CD8(+)perforin productivity,
2) CD3(-)CD161(+),
3) CD3(-)CD161(+)perforin productivity,
4) CD3(+)CD161(+),
5) CD3(+)CD161(+)perforin productivity.

2. The method for the assay according to claim 1, wherein the meaning of each assay is as follows:
1) the function which is meant by the assay of CD8(+)perforin productivity is an assay of CTL activity,
2) the function which is meant by the assay of CD3(-)CD161(+) is an assay of NK cell,
3) the function which is meant by the assay of CD3(-)CD161(+)perforin productivity is an assay of activation of NK cell,
4) the function which is meant by the assay of CD3(+)CD161(+) is an assay of NKT cell,
5) the function which is meant by the assay of CD3(+)CD161(+) perforin productivity is an assay of activation of NKT cell.

3. A method for a screening of a CTL activator comprising an assay of CD8(+)perforin productivity.

4. A novel β-1,3-glucan bearing a CTL activating ability obtained by the method for the screening of claim 3.

5. A CTL activator containing β-1,3-glucan obtained by the method for the screening of claim 3.

6. A method for a screening of an NK activator cpmprising an assay of CD3(-)CD161(+)perforin productivity.

7. A novel α-1,3-glucan bearing an NK activating ability obtained by the method for the screening of claim 6.

8. An NK activator containing an α-1,3-glucan obtained by the method for the screening of claim 6.

9. A method for an assay of kinetics of NK cells and NKT cells, **characterized in that**, CD3(-)CD161(+), CD3(-)CD161(+)perforin productivity, CD3(+)CD161(+) and CD3(+)CD161(+) perforin productivity of claim 1 are assayed at the same time.

10. A method for a fractional screening for an NK activator or an NKT activator comprising the method of claim 9 where activations of the NK cells and NKT cells are in a inverse correlation.

11. A novel α-1,3-glucan bearing an NK activating ability or an NKT activating ability obtained by the method for the screening of claim 10.

12. An NK activator or an NKT activator containing an α-1,3-glucan obtained by the method for the screening of claim 10.

13. A method for a calibration of correlation between the cancer species by the method for the assay of claim 9 and kinetics of NK cells and NKT cells.

14. An activator for NK cells or NKT cells containing α-1,3-glucan used in combination with at least one of anti-cancer agent, radioactivity and steroid therapy accompanied by the calibration of claim 13.

15. A test method for cancer in a sample of a cancer patient utilizing the information mentioned in any of claims 1 to 14.

16. A diagnostic method for cancer in a sample of a cancer patient utilizing the information mentioned in any of claims 1 to 14.

17. A therapeutic method for cancer in a sample of a cancer patient utilizing the information mentioned in any of claims 1 to 14.

18. A commercial medium where the information mentioned in any of claims 1 to 17 is carried on a medium utilizing the law of nature.

19. A commercial method utilizing the commercial medium of claim 18.
